(19) Europäisches Patentamt
European Patent Office
Office européen des brevets

(11) EP 1 991 259 B1

(12) EUROPEAN PATENT SPECIFICATION

(45) Date of publication and mention of the grant of the patent:
**10.10.2012 Bulletin 2012/41**

(21) Application number: **07706154.7**

(22) Date of filing: **15.02.2007**

(51) Int Cl.:
*A61K 39/00* (2006.01) *C07K 14/47* (2006.01)
*C12N 15/73* (2006.01)

(86) International application number:
**PCT/IL2007/000216**

(87) International publication number:
**WO 2007/094003 (23.08.2007 Gazette 2007/34)**

(54) **VIRAL DISPLAY VEHICLES FOR TREATING MULTIPLE SCLEROSIS**

VIRALE DISPLAY-VEHIKEL ZUR BEHANDLUNG VON MULTIPLER SKLEROSE

VÉHICULES D'AFFICHAGE DE VIRUS POUR TRAITER LA SCLÉROSE EN PLAQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.02.2006 US 773351 P**

(43) Date of publication of application:
**19.11.2008 Bulletin 2008/47**

(73) Proprietor: **Ramot at Tel-Aviv University Ltd.**
**61392 Tel Aviv (IL)**

(72) Inventors:
- **SOLOMON, Beka**
  **46399 Herzlia Pituach (IL)**
- **ZABAVNIK, Natalia**
  **75743 Rishon-lezion (IL)**
- **KOPPEL, Rela**
  **69122 Tel-aviv (IL)**
- **RAKOVER, Idan**
  **46447 Herzlia (IL)**

(74) Representative: **Raggers, René John et al**
**Exter Polak & Charlouis B.V.**
**P.O. Box 3241**
**2280 GE Rijswijk (NL)**

(56) References cited:
**WO-A2-02/074243**

- **SOMERS ET AL: "Profiling the autoantibody repertoire by serological antigen selection" JOURNAL OF AUTOIMMUNITY, LONDON, GB, vol. 25, no. 3, November 2005 (2005-11), pages 223-228, XP005166206 ISSN: 0896-8411**
- **CORTESE I ET AL: "IDENTIFICATION OF PEPTIDES SPECIFIC FOR CEREBROSPINAL FLUID ANTIBODIES IN MULTIPLE SCLEROSIS BY USING PHAGE LIBRARIES" PROCEEDINGS OF THE NATIONAL ACADEMY OF SCIENCES OF USA, NATIONAL ACADEMY OF SCIENCE, WASHINGTON, DC, US, vol. 93, October 1996 (1996-10), pages 11063-11067, XP002051334 ISSN: 0027-8424**
- **BAST G: "FOCIS abstract supplement" CLINICAL IMMUNOLOGY, ACADEMIC PRESS,, US, vol. 115, no. 1, April 2005 (2005-04), pages 3-282, XP004863152 ISSN: 1521-6616**
- **MIYAMOTO KATSUICHI ET AL: "The ICOS molecule plays a crucial role in the development of mucosal tolerance." JOURNAL OF IMMUNOLOGY (BALTIMORE, MD. : 1950) 1 DEC 2005, vol. 175, no. 11, 1 December 2005 (2005-12-01), pages 7341-7347, XP002434006 ISSN: 0022-1767**
- **DELMASTRO P ET AL: "Immunogenicity of filamentous phage displaying peptide mimotopes after oral administration" VACCINE, BUTTERWORTH SCIENTIFIC. GUILDFORD, GB, vol. 15, no. 11, August 1997 (1997-08), pages 1276-1285, XP004086601 ISSN: 0264-410X**
- **FRENKEL D ET AL: "Neuroprotection by IL-10-producing MOG CD4+ T cells following ischemic stroke" JOURNAL OF NEUROLOGICAL SCIENCES, ELSEVIER SCIENTIFIC PUBLISHING CO, AMSTERDAM, NL, vol. 233, no. 1-2, 15 June 2005 (2005-06-15), pages 125-132, XP004932292 ISSN: 0022-510X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **RAKOVER IDAN S ET AL: "Antigen-specific therapy of EAE via intranasal delivery of filamentous phage displaying a myelin immunodominant epitope.", JOURNAL OF NEUROIMMUNOLOGY 25 AUG 2010 LNKD-PUBMED:20546938, vol. 225, no. 1-2, 25 August 2010 (2010-08-25), pages 68-76, ISSN: 1872-8421**


Remarks:

The file contains technical information submitted after the application was filed and not included in this specification

**Description**

FIELD AND BACKGROUND OF THE INVENTION

The present invention relates to viral display vehicles displaying multiple sclerosis associated autoantigens which can be used to induce tolerance against auto-antigens associated with multiple sclerosis, and more particularly, to administration of such viral display vehicles for treating multiple sclerosis.

Multiple sclerosis (MS) is a chronic inflammatory disease of the central nervous system (CNS) affecting young adults (disease onset between 20 to 40 years of age), and the third leading cause for disability after trauma and rheumatic diseases. Disease prevalence is 120/100,000 and there are currently between 250,000 to 350,000 cases in North America. MS is characterized by a prominent infiltration of macrophages and T lymphocytes through the blood brain barrier (BBB) which induces active inflammation within the brain and spinal cord, attacking the myelin and resulting in gliotic scars, axonal loss and demyelination in the brain and spinal cord. Acute and chronic inflammatory processes characteristics to MS are visualized by brain and spinal cord MRI as hyperintense T2 or hypointense T1 lesions.

The etiology of MS is not fully understood. The disease develops in genetically predisposed subjects exposed to yet undefined environmental factors and the pathogenesis involves autoimmune mechanisms associated with autoreactive T cells against myelin antigens. MS is subdivided into several clinical subtypes; first signs involve onset of neurological symptoms affecting the CNS and accompanied by demyelinating lesions on brain magnetic resonance imaging (MRI). In 85 % of the patients the disease is characterized by a relapsing-remitting mode (RRMS). In about 15 % of patients the disease has a primary progressive course, characterized by gradual onset of neurological symptoms that progress over time, without any attacks. The only course of MS in which treatment was effectively established is RRMS. Thus, various immunomodulatory drugs such as interferon beta-1a .have been shown to reduce the number and severity of acute attacks, and decrease the accumulation of neurological disability. However, the overall efficacy of these drugs is limited to only 30-35 % of the cases.

The experimental model for MS; allergic encephalomyelitis (EAE), supports an autoimmune mechanism. In that model autoimmunity is mediated by CD4+ T cells which can be induced experimentally in susceptible strains of laboratory animals by immunization with CNS antigens, such as myelin basic protein (MBP) or proteolipid protein (PLP), as well as by adoptive transfer of activated CD4+ T cells specific for myelin antigens in appropriate adjuvant.

Analysis of the T cell reactivity to myelin antigens in MS patients revealed that the autoimmune response to myelin oligodendrocyte glycoprotein (MOG) predominates that of MBP, PLP or myelin-associated glycoprotein (MAG), and is directed against three main MOG epitopes; amino acids 1-22, 34-56 and 64-96 (Kerlero de Rosbo et aL 1997). Therefore, MOG is widely recognized as an important potential target antigen in MS pathogenesis.

MOG is a minor component of CNS myelin, exposed on the surface of the outermost lamellae of the myelin sheath. It is a glycoprotein consisting of 218-amino-acids (GenBank Accession No. AAA03180) with an extracellular Ig-like domain encompassing amino acids 1-125. Recent studies demonstrated that MOG is strongly encephalogenic in mice (Amor et al. 1994). Sun D., et al., (2001) demonstrated that administration of $MOG_{35-55}$ peptide results in enrichment of CD8+ $\alpha\beta$TCR+ T cells which when administered to mice cause encephalomyelitis via adoptive transfer. Sao H., et al. (2004) demonstrated the induction of optic neuritis (ON) and EAE by subcutaneous and footpad injections of $MOG_{35-55}$ and $MOG_{40-54}$ peptides.

Studies in experimental autoimmune models have shown the feasibility of inducing antigen-specific tolerance for disease resistance. Thus, intranasal administration of encephalitogenic epitopes of MBP was shown to protect against EAE induction (Bai et al. 1997). Other studies showed that antigen-specific tolerance is dependent on the route of administration and the amount of antigen used (Friedman and Weiner 1994). Thus, US Pat. No. 5,645,820 (to Hafler DA. and Weiner HL.) discloses aerosol or oral administration of auto-antigens such as MBP and type II collagen for the treatment multiple sclerosis and arthritis, respectively. However, due to their limited effect on disease symptoms (e.g., only a slight decrease in EAE scores from 3-4 to 2), such immunization modes are not clinically practiced.

Miyamoto et al. (2005, Journal of Immunology, vol. 175, no. 11, P 7341-7347) discloses that intranasal administration of MOG35-55 before EAE induction suppresses EAE and T cell responses.

U.S. Pat. No. 6,703,015 (to Solomon B. and Frenkel D.) discloses a display vehicle presenting a beta-amyloid epitope for treating Alzheimer's disease. When administered intraperitoneally or intranasally to a subject, the display vehicle was capable of eliciting antibodies against the beta-amyloid epitope and thus treat Alzheimer's disease. However, since these viral display vehicles resulted in production of endogenous antibodies against the displayed epitope, such display vehicles were never suggested for treating multiple sclerosis or any other autoimmune disease where auto-antibodies are deleterious and undesired.

There is thus a widely recognized need for, and it would be highly advantageous to have, methods and compositions for treating multiple sclerosis devoid of the above limitations.

SUMMARY OF THE INVENTION

According to one aspect of the present invention there is provided a composition-of-matter comprising a viral display vehicle displaying a multiple sclerosis associated antigen on a surface thereof.

According to another aspect of the present invention there is provided a pharmaceutical composition comprising, as an active ingredient, the composition-of-matter and a pharmaceutically acceptable carrier.

According to yet another aspect of the present invention there is provided a method of treating a multiple sclerosis, the method comprising administering to a subject in need thereof a therapeutically effective amount of the pharmaceutical composition, thereby treating the multiple sclerosis.

According to still another aspect of the present invention there is provided a use of the composition-of-matter for the manufacturing of a medicament identified for the treatment of multiple sclerosis.

According to further features in preferred embodiments of the invention described below, the pharmaceutically acceptable carrier is formulated for mucosal administration.

According to still further features in the described preferred embodiments administering is effected by trans-mucosal administration.

According to still further features in the described preferred embodiments administering is effected by intranasal administration.

According to still further features in the described preferred embodiments the medicament is formulated for trans-mucosal administration.

According to still further features in the described preferred embodiments the medicament is formulated for intranasal administration.

According to still further features in the described preferred embodiments the multiple sclerosis associated antigen comprises a MOG antigen.

According to still further features in the described preferred embodiments the MOG antigen comprises amino acids 37-44 of SEQ ID NO:18.

According to still further features in the described preferred embodiments the MOG antigen comprises an amino acid sequence selected from the group consisting of amino acids 1-22, 34-56, 64-49 and 35-55 of SEQ ID NO:18.

According to still further features in the described preferred embodiments the viral display vehicle comprises a filamentous bacteriophage.

According to still further features in the described preferred embodiments the filamentous bacteriophage is an fd bacteriophage.

According to still further features in the described preferred embodiments the filamentous bacteriophage comprises 150 copies of the antigen.

According to still further features in the described preferred embodiments the filamentous bacteriophage comprises 3000 copies of the antigen.

According to still further features in the described preferred embodiments the filamentous bacteriophage is selected from the group consisting of an M13 bacteriophage and an f1 bacteriophage.

According to still further features in the described preferred embodiments the multiple sclerosis associated antigen is selected from the group consisting of a myelin basic protein (MBP) antigen, a proteolipid protein (PLP) antigen, a myelin associated glycoprotein (MAG) antigen, a myelin-associated oligodendrocytic basic protein (MOBP) and an oligodendrocyte-specific protein (OSP).

According to still further features in the described preferred embodiments the therapeutically effective amount being capable of inducing immune tolerance.

The present invention successfully addresses the shortcomings of the presently known configurations by providing viral display vehicles displaying multiple sclerosis associated autoantigens which can be used for treating multiple sclerosis.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of".

The phrase "consisting essentially of’ or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

The term "method" refers to manners, means, techniques and procedures for accomplishing a given task including, but not limited to, those manners, means, techniques and procedures either known to, or readily developed from known manners, means, techniques and procedures by practitioners of the biotechnology and medical arts.

## BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1 is a schematic presentation depicting phage immunization protocols. Three groups of 8 weeks-old female C57BL/6 mice, were intranasally treated nine times with 25 μl of 5 x $10^{13}$ phages per ml phage constructs: phage alone; phage displaying MOG 150 copies (MOG 88), and phage displaying 3000 copies of MOG (MOG 8). The fourth group was treated with PBS. The first five administrations were performed prior to EAE induction every 3 days over a period of two weeks. Two weeks later, the mice were EAE induced (on days 30 or 31 of the experiment); on the next day the sixth administration was given. The following administrations were applied on day 45 (15 days after EAE induction; 7$^{th}$ administration), on day 114 (84 days after EAE induction; 8$^{th}$ administration) and on day 130 (100 days after EAE induction; the 9$^{th}$ administration) of the experiment. Bleedings of the mice were performed before the first immunization, after the fifth immunization, between the 7$^{th}$ and 8$^{th}$ immunization (one month after EAE induction) and after the 9$^{th}$ immunization (4 months after EAE induction).

FIGs. 2a-d depict EAE scores of mice subjected to the various immunization protocols depicted in Figure 1. Mice (three in each group) were immunized with either wild-type phage (which did not include the MOG antigen) (Figure 2b), phages expressing MOG f88 (150 copies of MOG antigen) (Figure 2c), phages expressing MOG f8 (3000 copies of MOG antigen) (Figure 2d) or PBS (Figure 2a), and following EAE induction the mice were observed for EAE induced phenotype using the EAE score test. Mice were observed daily for weight loss and clinical signs of EAE and scored on a scale 0-5 according to: 0, no disease; 1, limp tail; 2, hind limb weakness; 3, total hind leg or partial hind and front leg paralysis; 4, total hind leg and front leg paralysis; 5, moribund or dead. Note that while in mice treated with wild-type phage or PBS the EAE scores reached the levels of 3-5 even after 10 days of EAE induction, the EAE scores of mice treated with the MOG f88 (except for mouse 503) or the MOG f8 vectors remained as low as 0-1 for at least 150 days. Thus, these results demonstrate that immunization of mice with the viral display vehicle of the present invention which displays the MOG autoantigen is highly efficient in preventing EAE phenotype.

FIGs. 3a-b depict experimental design (Figure 3a) and EAE scores (Figure 3b) of mice treated with the phage MOG f88 displaying $MOG_{37-44}$. Mice were EAE induced using the $MOG_{35-55}$ emulsion and were further subjected to eight intranasal administrations (every three days) of either the phage MOG f88 (squares; f88 after induction) or PBS (triangles; EAE only). Note the significant effect of the viral display vehicle on ameliorating EAE induced symptoms (f88 after induction) as compared to the severe EAE symptoms (high EAE clinical scores) of mice subjected to EAE and administered with PBS alone (EAE only).

FIGs. 4a-c schematically depict maps of the phage display vectors of the present invention (Figures 4a-b) and a partial sequence alignment between the MOG-phage fusion constructs. Figure 4a - The fd-tet phage f8-1 vector displaying MOG37-44 peptide fused to all 3,000 copies of the major coat protein pVIII. Peptide was cloned into *Pst*I and *Bam*HI restricted sites of a single copy of pVIII gene; Figure 4b - The fd-tet phage f88-4 vector displaying MOG37-44 peptide fused to 150 copies of the pVIII by cloning into *Hind*III and *Pst*I sites of a duplicated copy of pVIII gene that is regulated by tac promoter. Figure 4c - Nucleic acid sequence alignment between partial sequences of MOG f8 (SEQ ID NO:22) and MOG f88 (SEQ ID NO:17) obtained from sequencing of positive clones containing the MOG epitope. The nucleic acid encoding the MOG epitope is set forth by (SEQ ID NO:12) and the encoded $MOG_{37-44}$ epitope amino acid sequence (VGWYRSPF) is set forth by SEQ ID NO:10.

## DESCRIPTION OF THE PREFERRED EMBODIMENTS

The present invention is of viral display vehicles displaying multiple sclerosis associated autoantigens on the surface thereof. Specifically, the present invention can be used to treat multiple sclerosis in a subject.

The principles and operation of the compositions and methods according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Multiple sclerosis (MS) is a chronic inflammatory disease of the central nervous system (CNS) characterized by a prominent infiltration of macrophages and T lymphocytes to the brain and spinal cord resulting in gliotic scars, axonal loss and demyelination in the brain and spinal cord. The disease develops in genetically predisposed subjects exposed to yet undefined environmental factors and the pathogenesis involves autoimmune mechanisms associated with autoreactive T cells against myelin antigens. Current therapy of MS involves the use of immunomodulatory drugs such as interferon beta-1a, however, such drugs have limited efficacy.

Attempts to treat MS by inducing tolerance against MS associated antigens include intranasal administration of encephalitogenic epitopes of MBP (Bai et al. 1997; Friedman and Weiner 1994; US Pat. No. 5,645,820 to Hafler DA. and Weiner HL.). However, due to the limited effect on disease symptoms (e.g., only a slight decrease in EAE scores from 3-4 to 2; US Pat. No. 5,645,820) such immunization modes are not clinically practiced.

The use of viral display vectors for immunization was demonstrated against diseases associated with formation of amyloid plaques such as Alzheimer's disease (U.S. Pat. No. 6,703,015 to Solomon B. and Frenkel D.). These vectors were designed to induce antibodies against a beta-amyloid epitope within the treated subject and thus treat Alzheimer's disease. However, since administration of the viral display vehicles resulted in production of endogenous antibodies against the displayed epitope, such display vehicles were never suggested for treating multiple sclerosis or any other autoimmune disease where auto-antibodies are deleterious and undesired.

While reducing the present invention to practice, the present inventors have uncovered a highly efficient method of treating multiple sclerosis by administering viral display vehicles displaying multiple sclerosis associated autoantigens on the surface thereof.

As described in Example 1 of the Examples sections which follows, the present inventors have constructed viral display vehicles of bacteriophage fd which display various copies of the $MOG_{37-44}$ amino acid sequence, a multiple sclerosis associated antigen. These compositions were able to prevent disease progression in a multiple sclerosis animal model (the EAE induced mouse) and to ameliorate symptoms of a full blown disease.

As is illustrated hereinbelow and in the Examples section which follows, while intraperitoneal administration of phage MOG f8 produced certain IgG antibodies titers, no IgG antibodies were observed following intranasal administration of the same phage. In addition, as is shown in Figures 1 and 2a-d and described in Example 1 of the Examples section which follows, serial intranasal immunizations of mice with either the MOG-f8 or MOG-f88 phages prior to and following EAE induction resulted in a complete abolishment of EAE phenotype for at least 150 days. In contrast, mice intranasally immunized with either PBS or an empty viral display vehicle (negative control) developed a sever EAE phenotype immediately after EAE induction (by subcutaneous injection with MOG35-55 peptide emulsified with incomplete Freund's adjuvant) which was maintained to various extents for at least 120 days. Moreover, as is shown in Figures 3a-b and described in Example 2 of the Examples section which follows, the MOG-f88 viral display vehicle was capable of ameliorating EAE-induced symptoms following disease onset. These results demonstrate the high therapeutic capacity of viral display vehicles displaying multiple sclerosis associated autoantigens to induce tolerance against the autoantigens and to treat multiple sclerosis.

Thus, according to one aspect of the present invention there is provided a composition-of-matter comprising a viral display vehicle displaying a multiple sclerosis associated autoantigen on a surface thereof.

As used herein the phrase "viral display vehicle" refers to any double stranded DNA viral particle, single stranded DNA viral particle or RNA viral particle capable of displaying the multiple sclerosis associated autoantigen of the present invention as a fusion protein of the virus coat protein.

Preferably, the viral display vehicle of the present invention comprises a filamentous bacteriophage. Such a filamentous phage is suitable for intranasal administration.

Non-limiting examples of viral display vehicles which can be used according to this aspect of the present invention include the fd bacteriophage, the M13 bacteriophage and the f1 bacteriophage.

The coat protein in which the autoantigen of the present invention is inserted (as a fusion protein) is preferably presented in multiple copies in each phage particle. Such a coat protein can be, for example, the major coat protein VIII (pVIII). Since the autoantigen is integrated in the viral coat protein, the number of copies of the coat protein reflects the number of copies of the autoantigen in each viral particle.

For example, as is shown in Example 1 of the Examples section which follows, the filamentous fd bacteriophage used by the present invention includes either 150 copies or 3000 copies of the coat protein VIII, thus, following ligation of the coding sequence encoding the autoantigen of the present invention into the coding sequence of the viral coat protein, the viral particle displays 150 or 3000 copies, respectively, of the autoantigen on the surface thereof.

Methods of constructing a viral display vehicle which displays the autoantigen of the present invention are well known in the art and are further described in the Example section which follows. Briefly, the coding sequence of the autoantigen of the present invention [e.g., SEQ ID NO:21 (5'-GTGGGGTGGTACCGCCCCCCCTTC-3') which encodes the human MOG37-44 epitope (SEQ ID NO:19) is ligated in-frame (using recombinant DNA technologies) into the genomic sequence of the viral coat protein such that following expression of the coat protein by the viral particle the autoantigen of the present invention is presented (displayed) on the coat protein.

For example, as described under the "General Materials and Experimental Methods" and Example 1 of the Examples section which follows, the nucleic acid sequence (SEQ ID NO:12; 5'- GTGGGCTGGTATCGCAGTCCGTTT -3') encoding the mouse multiple sclerosis antigen $MOG_{37-44}$ (SEQ ID NO:10) was ligated into the pVIII rec coat protein of the vector phage f8 [GenBank Accession No. AF218734 (SEQ ID NO:11)] or of the vector phage f88 [GenBank Accession No. AF218363 (SEQ ID NO:20)].

As used herein the term "autoantigen" refers to an amino acid sequence of an endogenous protein of a subject which is capable of eliciting an immune response in the subject.

Non-limiting examples of multiple sclerosis associated autoantigens include amino acid sequences of the myelin basic protein (MBP) (e.g., amino acids 84-103, 80-99, 83-99, 85-99 of GenBank Accession No. NP_001020272; SEQ ID NO:8), proteolipid protein (PLP) (e.g., amino acids 96-117, 106-125, 140-156 of GenBank Accession No. NP_ 000524.3; SEQ ID NO:15), myelin-associated glycoprotein (MAG) (e.g., amino acids 20-34, 124-137, 354-377, 570-582 of GenBank Accession No. NP_542167.1; SEQ ID NO:16), myelin-associated oligodendrocytic basic protein (MOBP) (e.g., amino acids 21-39, 15-36, of GenBank Accession No. NP_891980.1; SEQ ID NO:13), oligodendrocyte-specific protein (OSP) (e.g., amino acids 55-80, 179-207, 55-66 and 94-207 of GenBank Accession No. NP_005593.2; SEQ ID NO:14), and myelin oligodendrocyte glycoprotein (MOG) [e.g. amino acids 1-22, 34-56, 37-44 and 64-96 of SEQ ID NO: 7 (mouse MOG; GenBank Accession No. AAA03180); which correspond to amino acids 1-22, 34-56, 37-44 and 64-96 of SEQ ID NO:18 (human MOG; GenBank Accession No. AAB08090), respectively [for MOG homology between human and mouse see Johns, T.G. and C.C. Bernard (1999), The structure and function of myelin oligodendrocyte glycoprotein. J. Neurochem. 72 (1): 1-9].

Preferably, the multiple sclerosis associated antigen used by the present invention comprises amino acids 37-44 of SEQ ID NO:18 (VGWYRPPF; SEQ ID NO:19).

Multiple sclerosis associated autoantigens can be identified from a plurality of synthetic peptides derived from a candidate protein (e.g., MOBP). Such peptides, can be, for example, overlapping peptides of 10-20 amino acids which are preferably emulsified in complete Freund's adjuvant (CFA) and further administered (e.g., intraperitoneally) into a multiple sclerosis animal model [the allergic encephalomyelitis (EAE) animal model; C57bl/6 mice (Shao H, Huang Z, Sun SL, Kaplan HJ, Sun D. Myelin/oligodendrocyte glycoprotein-specific T-cells induce severe optic neuritis in the C57BL/6 mouse. Invest Ophthalmol. Vis. Sci. 2004, 45: 4060-5)]. The effect of the administered peptides on EAE symptoms (as assessed using EAE acceptable scores, see e.g., the Examples section which follows) is evaluated and the candidate autoantigens are those peptides resulting in relatively high EAE scores (e.g., scores of 4-5), essentially as described in Holz A, et al., 2000 [J. Immunol. 164(2):1103-9]. Candidate peptides are further qualified for their ability to induce an immune response in multiple sclerosis patients using, for example, a lymphocyte proliferation assay with lymphocytes obtained from multiple sclerosis patients [see for example, Holz, 2000 (Supra)].

It will be appreciated that the viral display vehicles which display the multiple sclerosis associated autoantigens of the present invention can be used to treat multiple sclerosis.

As used herein the phrase "treating" refers to inhibiting, preventing or arresting the development of a pathology (*i.e.*, multiple sclerosis) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein, the term "subject" (or "individual" which is interchangeably used herein) refers to an animal subject e.g., a mammal, e.g., a human being at any age who is diagnosed with or is at risk of developing the pathology. Non-limiting examples of individuals who are at risk to develop the pathology of the present invention include individuals who are genetically predisposed to develop the pathology (e.g., individuals who carry a mutation or a DNA polymorphism which is associated with high prevalence of the pathology), and/or individuals who are at high risk to develop the pathology due to presence of similar pathologies or other factors such as environmental hazard. For example, an individual who is diagnosed with a certain autoimmune disease (e.g., type I diabetes mellitus) is at higher risk of developing multiple sclerosis (see Janice S. et al., Type 1 Diabetes and Multiple Sclerosis: Together at last, Diabetes Care 26:3192-3193, 2003).

Thus, according to another aspect of the present invention there is provided a method of treating multiple sclerosis. The method is effected by administering to a subject in need thereof a therapeutically effective amount of the composition-of-matter of the present invention (the viral display vehicle which displays the multiple sclerosis associated autoantigen of the present invention), thereby treating multiple sclerosis.

As used herein a "therapeutically effective amount" refers to an amount of the composition-of-matter of the present invention (the viral display vehicle which displays the multiple sclerosis associated autoantigen of the present invention) which is capable of the biological effect (treating multiple sclerosis). Preferably, the therapeutically effective amount of the composition-of-matter of the present invention is selected such that it is capable of inducing immune tolerance against the autoantigens associated with multiple sclerosis while avoiding endogenous antibody production against the displayed autoantigens.

Thus, the viral display vehicle of the present invention which displays the multiple sclerosis associated autoantigens is preferably administered to oral or mucosal tissues where it is capable of inducing immune tolerance while avoiding production of autoantigens against multiple sclerosis. In case viral amplification within the subject (e.g., bacterial mediated) is less desired, measures may be taken to avoid contact with the natural flora (e.g., by mode of administration) or propagation therewith (e.g., UV radiated particles).

Oral and mucosal tolerance for suppression and treatment of autoimmune disease is known in the art. For example, Weiner et al. have disclosed therapy, for the treatment of rheumatoid arthritis by mucosal administration of collagen and collagen peptides (US Patent Nos. 5,399,347; 5,720,955; 5,733,542; 5,843,445; 5,856,446; and 6,019,975), treatment of Type I diabetes by mucosal administration of insulin (5,643,868; 5,763,396; 5,843,445; 5,858,968; 6,645,504; and 6,703,361) or glucagon (6,645,504), uveoretinitis by mucosal administration of toleragens (5,961,977), and multiple sclerosis by mucosal administration of myelin basic protein (MBP) (5,849,298; 5,858,364; 5,858,980; 5,869,093; 6,077,509). Additional candidate conditions, antigens and modes of treatment by mucosal tolerance have been disclosed in US Patent Nos. 6,812,205, 5,935,577; 5,397,771; 4,690,683 to Weiner et al., US Patent No. 6,790,447 to Wildner et al; International Patent Nos. EP 0886471 A1, WO 01821951 to Haas, et al, US Patent No. 5,843,449 to Boots et al. (HCgp-39 for arthritis), and US Patent No: 2004 0029 786 to Das (mucosal tolerance and relief from Crohn's disease by administration of Colonic Epithelial Protein).

Induction of mucosal tolerance (e.g., using trans-mucosal administration) according to the invention is an advantageous method for treating multiple sclerosis for several reasons:

(1) Absence of toxicity: no toxicity has been observed in clinical trials or animal experiments involving oral or other mucosal administration of protein antigens, such as bovine myelin [which contains myelin basic protein (MBP) and proteolipid protein (PLP)] to humans afflicted with multiple sclerosis, or oral or by-inhalation administration of chicken Type II collagen to humans or rodents afflicted with rheumatoid arthritis [or a corresponding animal model disorder]; or oral administration of bovine S-antigen to humans afflicted with uveoretinitis; or oral administration of insulin to healthy volunteers.

(2) Containment of immunosuppression. Conventional treatments of immune system disorders involve administration of non-specific immunosuppressive agents, such as the cytotoxic drugs methotrexate, cyclophosphamide (CYTOXAN$^{RTM}$, Bristol-Myers Squibb), azathioprine (IMURAN$^{RTM}$, Glaxo Wellcome) and cyclosporin A (SANDIMMUNE$^{RTM}$, NEORAL$^{RTM}$, Novartis). Steroid compounds such as prednisone and methylprednisolone (also non-specific immunosuppressants) are also employed in many instances. All of these currently employed drugs have limited efficacy (e.g., against both cell-mediated and antibody-mediated autoimmune disorders). Furthermore, such drugs have significant toxic and other side effects and, more important, eventually induce "global" immunosuppression in the subject being treated. Prolonged treatment with the drugs down-regulates the normal protective immune response against pathogens, thereby increasing the risk of infection. In addition, patients subjected to prolonged global immunosuppression have an increased risk of developing severe medical complications from the treatment such as malignancies, kidney failure and diabetes.

(3) Convenience of therapy. Mucosal administration is more convenient than parenteral, or other forms, of administration.

(4) Greatly reduced incidence of alteration of the tolerizing molecule by digestive and metabolic processes (especially in non-oral routes of administration). These advantages provide superior protection from atherogenic processes, improved patient compliance and reduced cost of therapy.

Without being bound to any theory, induction of tolerance via oral or mucosal administration can result from interaction between the tolerizing autoantigen displayed on the viral display vehicle of the present invention and the mucosal associated lymphatic tissue (MALT), allowing the accumulation of tolerizing amounts of the autoantigen in the MALT.

Thus, induction of tolerance against the multiple sclerosis associated autoantigen is preferably performed by administering the composition-of-matter of the present invention to a mucosal surface of the subject.

As used herein, the phrase "mucosal surface" is defined as a portion of the anatomy having exposed mucosal membranes having component or components of the mucosal associated lymphatic tissue. As used herein, the phrase "mucosal administration" is defined as application of the composition-of-matter of the present invention to at least one mucosal surface. Non-limiting examples of mucosal administration are buccal, intranasal, otic (middle ear), conjunctival, vaginal, rectal, eye, etc. Mucosal administration excludes, for example, intravenous, subcutaneous and epidural administration.

It will be appreciated that the composition-of-matter of the present invention (which includes the viral display vehicle of the present invention which displays the autoantigen of the present invention) can be administered to the subject per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the composition-of-matter of the present invention (the viral display vehicle of the present invention which displays the multiple sclerosis associated autoantigen of the present invention) accountable for the biological effect (treating multiple sclerosis).

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable formulations according to the invention include formulations of the composition-of-matter of the present invention adapted for oral, enteral, buccal, nasal, bronchial or intrapulmonary administration. The preparation of such formulations is well within the skill of the art. Thus, it is preferred that such formulations not contain substances that can act as adjuvants in order to avoid sensitization of the treated subject.

Suitable oral formulations for use according to the present invention can be in any suitable orally administrable form, for example, a pill, a liquid, or a capsule or caplet containing an effective amount of the autoantigen. Each oral formulation may additionally comprise inert constituents including pharmaceutically acceptable carriers, diluents, fillers, disintegrants, flavourings, stabilizers, preservatives, solubilizing or emulsifying agents and salts as is well-known in the art. For example, tablets may be formulated in accordance with conventional procedures employing solid carriers and other excipients well-known in the art. Capsules may be made from any cellulose derivatives. Non-limiting examples of solid carriers include starch, sugar, bentonite, silica and other commonly used inert ingredients. Diluents for liquid oral formulations can include inter alia saline, syrup, dextrose and water.

The composition-of-matter of the present invention (which includes the viral display vehicle displaying the autoantigen of the present invention) can also be made up in liquid formulations or dosage forms such as, for example, suspensions or solutions in a physiologically acceptable aqueous liquid medium. Such liquid media include water, or suitable beverages, such as fruit juice or tea which will be convenient for the patient to sip at spaced apart intervals throughout the day. When given orally in liquid formulations the composition-of-matter of the present invention may be dissolved or suspended in a physiologically acceptable liquid medium, and for this purpose the composition-of-matter of the present invention may be solubilized or adjusted to a pH within physiologically acceptable limits (e.g., 3.5 to 8).

Sustained release oral delivery systems are also contemplated and are preferred. Non-limiting examples of sustained release oral dosage forms include those described in U.S. Pat. No. 4,704,295, issued Nov. 3, 1987; U.S. Pat. No. 4,556,552, issued Dec. 3, 1985; U.S. Pat. No. 4,309,404, issued Jan. 5, 1982; U.S. Pat. No. 4,309,406, issued Jan. 5, 1982; U.S. Pat. No. 5,405,619, issued Apr. 10, 1995; PCT International Application WO 85/02092, published May 23, 1985; U.S. Pat. No. 5,416,071, issued May 16, 1995; U.S. Pat. No. 5,371,109, issued Dec. 6, 1994; U.S. Pat. No. 5,356,635, issued Oct. 18, 1994; U.S. Pat. No. 5,236,704, issued Aug. 17, 1993; U.S. Pat. No. 5,151,272, issued Sep. 29, 1992; U.S. Pat. No. 4,985,253, issued Jan. 15, 1991; U.S. Pat. No. 4,895,724, issued Jan. 23, 1990; and U.S. Pat. No. 4,675,189, issued Jun. 23, 1987.

Sustained release oral dosage forms coated with bioadhesives can also be used. Examples are compositions disclosed in European Published Application EP 516141; U.S. Pat. No. 4,226,848; U.S. Pat. No. 4,713,243; U.S. Pat. No. 4,940,587; PCT International Application WO 85/02092; European Published Application 205282; Smart J D et al. (1984) J Pharm Pharmacol 36:295-9; Sala et al. (1989) Proceed Intern Symp Control Rel Bioact Mater 16:420-1; Hunter et al. (1983) International Journal of Pharmaceutics 17:59-64; "Bioadhesion-Possibilities and Future Trends, Kellaway," Course No. 470, May 22-24, 1989.

Commercially available sustained release formulations and devices include those marketed by ALZA Corporation, Palo Alto, Calif., under tradename ALZET, INFUSET, IVOS, OROS, OSMET, or described in one or more U.S. Pat. No. 5,284,660, issued Feb. 9, 1994; U.S. Pat. No. 5,141,750, issued Aug. 25, 1992; U.S. Pat. No. 5,110,597, issued May 5, 1992; U.S. Pat. No. 4,917,895, issued Apr. 17, 1990; U.S. Pat. No. 4,837,027, issued Jun. 6, 1989; U.S. Pat. No. 3,993,073, issued Nov. 23,1976; U.S. Pat. No. 3,948,262, issued Apr. 6,1976; U.S. Pat. No. 3,944,064, issued Mar 16 1976; and U.S. Pat. No. 3,699,963; International Applications WO9511011 and WO9515191; and European Published Applications EP 259013 and EP 354742.

Orally administrable pharmaceutical formulations containing the composition-of-matter of the present invention are prepared and administered to mammals who have manifested symptoms of multiple sclerosis. Additionally, subjects who are at risk for developing multiple sclerosis, e.g., having a genetic predisposition to developing the disorder, as determined through suitable means, such as genetic studies and analysis, are treated with similar oral preparations.

Pharmaceutical formulations for oral or enteral administration to treat multiple sclerosis are prepared from the composition-of-matter of the present invention and a pharmaceutically acceptable carrier suitable for oral ingestion.

For by-inhalation administration (i.e., delivery to the bronchopulmonary mucosa) suitable sprays and aerosols can be used, for example using a nebulizer such as those described in U.S. Pat. No. 4,624,251 issued Nov. 25, 1986; U.S. Pat. No. 3,703,173 issued Nov. 21, 1972; U.S. Pat. No. 3,561,444 issued Feb. 9, 1971; and U.S. Pat. No. 4,635,627 issued Jan. 13, 1971. The aerosol material is inhaled by the subject to be treated.

Other systems of aerosol delivery, such as the pressurized metered dose inhaler (MDI) and the dry powder inhaler as disclosed in Newman S P in Aerosols and the Lung, S W Clarke S W and D Davis, eds. pp. 197-224, Butterworths, London, England, 1984, can be used when practicing the present invention.

Aerosol delivery systems of the type disclosed herein are available from numerous commercial sources including Fisons Corporation (Bedford, Mass.), Schering Corp. (Kenilworth, N.J.) and American Pharmoseal Co. (Valencia, Calif.).

Formulations for nasal administration can be administered in an aqueous solution. Preferred aerosol pharmaceutical formulations may comprise for example, a physiologically acceptable buffered saline solution containing the composition-of-matter of the present invention.

Specific non-limiting examples of the carriers and/or diluents that are useful in the pharmaceutical formulations of the present invention include water and physiologically acceptable buffered saline solutions such as phosphate buffered saline solutions pH 7.0-8.0.

The mucosally administered formulation of the present invention may include a thermosetting gel which increases in viscosity at body temperature upon contact with the mucosa.

Formulations for buccal administration can include mucoadhesive mixed with effective amounts of the composition-of-matter of the present invention. Effective amounts are anticipated to vary according to the formulation employed. For formulation administered by inhalation, the effective amount is likely to be less than that of the oral dose.

The treatment may be discontinued if desired (in the judgment of the attending physician) and the patient monitored for signs of relapse. If clinical symptoms or other disorder indicators show that the patient is relapsing, treatment may resume.

As will be understood by those skilled in the art, the dosage will vary with the various compositions-of-matter of the present invention and may vary with the sex, age, and physical condition of the patient as well as with other concurrent treatments being administered. Consequently, adjustment and refinement of the dosages used and the administration schedules will preferably be determined based on these factors and especially on the patient's response to the treatment. Such determinations, however, require no more than routine experimentation, as illustrated in Examples provided below.

Determination of a therapeutically effective amount is well within the capability of those skilled in the art, especially in light of the detailed disclosure provided herein.

For any preparation used in the methods of the invention, the toxicity, therapeutically effective amount or dose can be estimated initially from *in vivo* animal models. The data obtained from these in vitro or animal studies can be used in formulating a range of dosage for use in human. For example, a dose can be formulated in animal models [e.g., the EAE mouse model for multiple sclerosis, C57bl/6 mice (Shao H, Huang Z, Sun SL, Kaplan HJ, Sun D. Myelin/oligodendrocyte glycoprotein-specific T-cells induce severe optic neuritis in the C57BL/6 mouse. Invest Ophthalmol Vis Sci. 2004, 45: 4060-5] to achieve a desired concentration or titer. Such information can be used to more accurately determine useful doses in humans. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient (the composition-of-matter of the present invention) which are sufficient to induce tolerance against the autoantigen and treat multiple sclerosis (minimal effective concentration, MEC). The MEC will vary for each preparation, but can be estimated from in vitro/in vivo data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

It will be appreciated that the composition-of-matter of the present invention can be provided to the individual along with other known multiple sclerosis agents such as interferon beta-1a in order to increase the therapeutic effect thereof. However, measures are taken to avoid cross-effectiveness and unwanted side effects.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

Compositions of the present invention may be included in an article-of-manufacture, packaged and identified for use in the treatment of multiple sclerosis in a subject in need thereof. The article-of-manufacture includes a packaging material and the composition-of-matter of the present invention. The packaging material including a label or package insert indicating that the composition-of-matter of the present invention is for treating multiple sclerosis.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual." CSHL Press (1996); all of which are incorporated by reference as if fully set forth herein. Other general references are provided throughout this document. The procedures therein are believed to be well known in the art and are provided for the convenience of the reader.

### *GENERAL MATERIALS AND EXPERIMENTAL METHODS*

#### *Construction of a phage vector displaying the MOG 37-44 amino acid sequence (SEQ ID NO:10)*

***Preparation** of **competent E. Coli cells -*** E. coli K91 Kan were grown in 2 ml of 2YT media containing 100 μg/ml Kanamycin at 37 °C while shaking (250 RPM) overnight. Then, 500 μl of overnight culture was transferred to 100 ml SOB media grown to an early logarithmic phase [O.D. (600 nm) ~ 0.3]. The grown bacteria were incubated on ice for 10 minutes and then centrifuged at 5000 rpm for 10 minutes at 4 °C. The pellet was resuspended in 20 ml CCMB and incubated for 20 minutes on ice followed by 10 minutes centrifugation at 5000 rpm at 4 °C. The precipitant bacteria were resuspended in 4 ml CCMB and were kept on ice while divided into aliquots and stored at -70 °C.

***Cloning of phage MOG f8 and MOG f88***

***F8-1 vector purification and cleavage*** - The vector f8-1 was purified from a "type 8" fd bacteriophage library clone using a Qiagen DNA purification kit [Smith GP, Scott JK (1993) Libraries of peptides and proteins displayed on filamentous phage. Methods Enzymol 217:228-257; Smith GP (1991) Surface presentation of protein epitopes using bacteriophage expression systems. Curr Opin Biotechnol 2:668-673]. The vector was digested by two *Pst*I and *Bam*HI restriction enzymes separately. First, a total reaction volume of 40 μl containing 15 μg of dsDNA, 1 μl *Pst*I, 4 μl of a suitable restriction enzyme buffer was incubated for 1 hour at 37 °C, followed by inactivation at 70 °C for 5 minutes. *Bam*HI (1 μl) was then added to the reaction mixture and was further incubated for 1 hour at 37 °C followed by inactivation at 70 °C for 5 minutes. In order to remove the 5' phosphate groups from the digested vector, 1 μl of Antarctic Phosphatase was added for a 30-minutes incubation at 37 °C. The phosphatase treated DNA was then applied onto 0.7 % agarose gel electrophoresis. The linearized vector was purified from gel using Qiagen DNA extraction kit

***F88-4 vector purification and cleavage*** - Purification and digestion of f88-4 vector, "type 88" was performed similarly to that described above (Smith, 1991, 1993, Supra), the difference being that *Hind*III and *Pst*I were added together for 2 hour incubation at 37 °C. Then the vector was de-phosphorylated and purified as explained above.

***Insert preparation: Phosphorylation of insert primers and annealing -*** Two complementary primers were synthesized in Sigma, each primer separately; 5 μl of 10 pmol/μl was mixed with 2.5μl T4 kinase bufferX10, 1 μl T4 kinase and 16.5 μl double distilled waster (DDW) followed by 2 hours incubation at 37 °C subsequently inactivated for 10 minutes at 70 °C. Then, 20 pmol of each primer were added and incubated together for 5 minutes at 95 °C, after which the temperature was gradually lowered. For the f8-1 vector, annealed insert primers [Forward primer (SEQ ID NO:1): 5'-GAGGTGGGCTGGTATCGCAGTCCGTTTGAG-3'; reverse primer (SEQ ID NO:2): 5'-GATCCTCAAACGGACTGCGATACCAGCCCACCTCTGCA-3'] results in dsDNA with *Pst*I and *Bam*HI sticky ends, and for the f88-4 annealed insert primers [Forward primer (SEQ ID NO:3): 5'-AGCTTTGCCGAGGTGGGCTGGTATCGCAGTCCGTTTATTGCA-3' ; reverse primer (SEQ ID NO:4): 5'-ATAAACGGACTGCGATACCAGCCCACCTCGGCAA-3'] results in dsDNA with *Hind*III and *Pst*I sticky ends (see vector maps in Figures 4a-b).

***Ligation and transformation -*** Purified vector and annealed primers (insert) were ligated at a ratio of 1:3 respectively, as follows: 100 ng of vector, 1 ng insert, 1.5 μl DNA ligase buffer X10, 1 μl DNA ligase, incubated overnight at 16 °C. The ligation products were transferred into competent K91Kan using heat shock method. 10 μl of ligation was added to 100 μl of bacteria and the mix was kept on ice for 30 minutes, followed by 2 minutes incubation at 42 °C then returned to ice. 2YT (1 ml) was added and bacteria were incubated by shaking at 150 rpm for 1.5 hours at 37 °C to express antibiotic resistant genes. Transformed cells were grown at 37 °C on 2YT plates containing 100 μg/ml Kanamycin and 20μg/ml Tetracycline overnight.

***Identification of positive clones -*** A number of colonies that had grown on plates were screened for the presence of insert using colony PCR. Each colony was mixed with 7 μl of ready mix, 5 μl sterile DDW, and 1 μl (10 pmol) of each primer and subjected to PCR reaction. The primers used were as follows: The forward primer of the insert had served as forward primer (*i.e.*, SEQ ID NO:1 served as a forward primer for identification of positive clones in f8 vector and SEQ ID NO:3 and served as a forward primer for identification of positive clones in f88 vector) and the reverse primer was complementary either to pVIII of f8-1 vector (5'-CAGCTTGCTTTCGAGATGA-3'; SEQ ID NO:5) or the f88-4 vector (5'-AGTAGCAGAAGCCTGAAGA-3'; SEQ ID NO:6). The PCR products were then applied to a 2 % agarose gel in order to detect 170 bp bands indicating that the MOG insert is on the plasmid. Positive colonies were then sequenced using reverse primer that was used in PCR. Figure 4c depicts partial sequence alignment of positive clones.

***Scale-up of phage production -*** The cloned E. coli K91Kan containing vector f8 with MOG insert were grown in 500 ml of 2YT media, including 100 μg/ml Kanamycin and 20 μg/ml Tetracycline overnight at 37 °C, to which K91Kan with vector f88 MOG with an addition of 2 mM IPTG was supplied. The next day, the inoculum was centrifuged at 6500 rpm at 4 °C for 20 minutes in order to eliminate bacteria presence. Supernatant was collected and incubated with PEG/ NaCl in 5:1 (v/v) ratio overnight at 4 °C which enables phage precipitation. Phages were then precipitated by one hour centrifugation at 9000 rpm at 4 °C. After supernatant was discarded, the precipitate was resuspended in 20 ml of sterile PBS and precipitated

$$\frac{O.D._{(269nm)} - O.D._{(320nm)} * 6 * 10^{1'}}{9273} = \text{phages/ml}$$

once again by overnight incubation with PEG/NaCl at ratio 1:5 at 4 °C. Phage precipitation was achieved by a one hour centrifugation at 9000 rpm at 4 °C, resuspended in 1 ml sterile PBS and then filtrated using 0.45 μm filter tip to eliminate any traces of bacteria. The recovered phage concentration was determined according to its absorbance at 269 nm and 320 nm as reference, measured by spectrophotometer, according to the formula:

***EAE induction in mice*** - Female C57BL/6 mice, 10-weeks old, were immunized with myelin oligodendrocyte glycoprotein peptide [MOG35-55; MEVGWYRSPFSRVVHLYRNGK (SEQ ID NO:9)] 150 μg/200 μl, synthesized by PEPTIDES international company, Louisville, Kentucky, purified to 98.6 % by HPLC. MOG35-55 peptide was dissolved in 100 μl double distilled water (DDW) and emulsified with additional 100 μl incomplete Freund's adjuvant (IFA) (Sigma-Aldrich, St. Louis, MO) containing 500 ng heat-inactivated H37Ra Mycobacterium tuberculosis (Difco, Detroit, MI,USA). A total of 200 μl MOG emulsion was subcutaneously injected into four sites on the flanks of mice near the tail. At days 0 and 1 of post-immunization, mice received additional injections (intraperitoneally) of Pertussis Toxin (Sigma, Deisenhofen, Germany), 500 ng/300 μl PBS.

***EAE scoring system -*** The phenotype of EAE induction was scored on a scale of 0-5 according to: "0", no disease; "1", limp tail; "2", hind limb weakness; "3", total hind leg or partial hind and front leg paralysis; "4", total hind leg and front leg paralysis; "5", moribund or dead.

***EXAMPLE 1***

***INTRANASAL ADMINSITRATION OF A VIRAL DISPLAY VEHICLE DISPLAYING MOG A UTOANTIGENS PREVENTS EAE-INDUCED PHENOTYPE***

To test whether intranasal administration of a viral display vehicle displaying a MOG peptide can induce tolerance against multiple sclerosis associated autoantigens, the present inventors have challenged C57BL/6 mice with phage MOG-f8 or MOG-f88, as follows.

***Experimental Results***

***Construction of viral display vehicles displaying the MOG37-44 epitope (VGWYRSPF; SEQ ID NO:10)*** - The present inventors have genetically engineered a recombinant fd phage, displaying at its surface a chimeric pVIII major coat protein fused to the $MOG_{37-44}$ amino acid sequence (SEQ ID NO:10) which is part of the previously identified encephalogenetic peptide $MOG_{35-55}$ (MEVGWYRSPFSRVVHLYRNGK; SEQ ID NO:9). The recombinant phages MOG-f8 and MOG-f88 displayed 3000 or 150 copies of the $MOG_{37-44}$ epitope, respectively. Expression of the $MOG_{37-44}$ epitope was measured by testing the reactivity of bacteriophages to polyclonal antibodies against peptide $MOG_{35-55}$ (SEQ ID NO:9). The positive results demonstrated that the recombinant phages displayed the 37-44 amino acid sequence of MOG on their surface.

***Intranasal administration of phage MOG-f8 resulted in no IgG antibodies -*** In animals that received five doses of phage MOG-f8 intranasally, no IgG antibodies were detected against $MOG_{35-55}$, while intraperitoneal administration of mice produced certain titers against $MOG_{35-55}$.

***Viral display vehicle displaying multiple sclerosis associated MOG autoantigen is capable of preventing development of EAE disease -*** The next step was to evaluate the ability of viral display vehicle of the present invention which displays the MOG37-44 antigen to prevent development of EAE disease. Briefly, 8 weeks-old female C57BL/6 mice were intranasally treated eight times with phage displaying 3000 copies of MOG (MOG 8) (25 μl of 5 x $10^{13}$ phages / ml) or phage displaying 150 copies of MOG (MOG 88) (25 μl of 5 x $10^{13}$ phages / ml). The first five treatments were given every 3 days (for a period of two weeks) prior to EAE induction. Two weeks later the mice were EAE induced using the MOG35-55 emulsion. The following four administrations were applied after 1 day ($6^{th}$ administration), 2 weeks ($7^{th}$ administration), 2.5 months ($8^{th}$ administration) and 3.5 months ($9^{th}$ administration) (Figure 1). The mice were observed daily for clinical signs of EAE. As shown in Figures 2a-d, phage treatment before and after EAE induction prevented the disease progression.

These results demonstrate that intranasal administration of mice with the viral display vehicle of the present invention (e.g., phage MOG f8) is highly efficient in preventing EAE phenotype. Thus, these results suggest that intranasal administration of the viral display vehicle of the present invention which display a multiple sclerosis associated autoantigen (e.g., human $MOG_{37-44}$ as set forth by SEQ ID NO:19) can be used for preventing multiple sclerosis symptoms and thus treating multiple sclerosis.

***EXAMPLE 2***

***TREATING OF EAE-IND UCED MICE USING THE VIRAL DISPLAY VEHICLE DISPLAYING A MOG A UTOANTIGEN***

To further evaluate the capacity of the viral display vehicle of the present invention (which displays a multiple sclerosis associated antigen, e.g., $MOG_{37-44}$), the present inventors have intranasally administered the viral display vehicle to mice which were subjected to EAE induction with the MOG35-55 emulsion, as follows.

Eight weeks-old female C57BL/6 mice were subjected to EAE induction using the MOG35-55 emulsion and following EAE induction the mice were intranasally treated eight times with phage displaying 150 copies of MOG (MOG 88) (25 $\mu$l of 5 x $10^{13}$ phages / ml). Intranasal administrations of the MOG 88 were performed on days 3, 6, 9, 12, 15, 18, 21 and 24 following EAE induction (Figure 3a). The mice were observed daily for clinical signs of EAE. As is shown in Figure 3b, intranasal administration of the MOG 88 phage after EAE induction resulted in drastic amelioration of disease symptoms.

These results demonstrate that intranasal administration of mice with the viral display vehicle of the present invention which displays a multiple sclerosis associated autoantigen (e.g., phage MOG f88) is highly efficient in treating EAE symptoms. Thus, these results suggest the use of a viral display vehicle displaying a multiple sclerosis associated autoantigen such as human MOG37-44 (SEQ ID NO:19) for treating multiple sclerosis after disease onset (*i.e.*, after multiple sclerosis was diagnosed in the subject).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

In addition, citation or identification of any reference in this application shall not be construed as an admission that such reference is available as prior art to the present invention.

***REFERENCES***

***(Additional references are cited in text)***

1. Amor, S., N. Groome, C. Linington, et al. (1994). "Identification of epitopes of myelin oligodendrocyte glycoprotein for the induction of experimental allergic encephalomyelitis in SJL and Biozzi AB/H mice." J Immunol 153(10): 4349-4356;
2. Bai, X.-F., F.-D. Shi, B.-G. Xiao, et al. (1997). "Nasal administration of myelin basic protein prevents relapsing experimental autoimmune encephalomyelitis in DA rats by activating regulatory cells expressing IL-4 and TGF-[beta] mRNA." Journal ofNeuroimmunology 80(1-2):65-75;
3. Friedman, A. and H. L. Weiner (1994). "Induction of Anergy or Active Suppression Following Oral Tolerance is Determined by Antigen Dosage." PNAS 91(14): 6688-6692;
4. Kerlero de Rosbo, N., M. Hoffman, I. Mendel, et al. (1997). "Predominance of the autoimmune response to myelin oligodendrocyte glycoprotein (MOG) in multiple sclerosis: reactivity to the extracellular domain of MOG is directed against three main regions." Eur J Immunol 27(11): 3059-69;
5. Pham-Dinh, D., M. Mattei, J. Nussbaum, et al. (1993). "Myelin/Oligodendrocyte Glycoprotein is a Member of a Subset of the Immunoglobulin Superfamily Encoded within the Major Histocompatibility Complex." PNAS 90(17): 7990-7994;
6. Parkman, Graft-versus-host Disease, Ann. Rev. Med., 1991, 42: 189-197;
7. Sao H., et al., 2004. Invest. Ophthalmol. Vis. Sci. 45: 4060-4065;
8. Sun D., et al., 2001. The Journal of Immunology 166: 7579-7587;

## Claims

1. Filamentous bacteriophage displaying a MOG antigen comprising amino acids 37-44 of SEQ ID NO:18 fused to a major coat protein VIII on a surface thereof.

2. Filamentous bacteriophage according to claims 1, wherein said filamentous bacteriophage is selected from the group consisting of a fd bacteriophage, M13 bacteriophage and f1 bacteriophage.

3. Filamentous bacteriophage according to claims 1 -2, wherein said filamentous bacteriophage is a fd bacteriophage.

4. Filamentous bacteriophage according to any of the claims 1-3, wherein said filamentous bacteriophage comprises 150 copies of said MOG antigen.

5. Filamentous bacteriophage according to any of the claims 1-4, wherein said filamentous bacteriophage comprises 3000 copies of said MOG antigen.

6. Pharmaceutical composition for nasal administration comprising, as an active ingredient, the filamentous bacteriophage according to any of the claims 1-5, and a pharmaceutically acceptable carrier.

7. Filamentous bacteriophage displaying a MOG antigen comprising amino acids 37-44 of SEQ ID NO:18 fused to a major coat protein VIII on a surface thereof for use in treatment of multiple sclerosis by intranasal administration.

8. Filamentous bacteriophage for use in the treatment of multiple sclerosis by intranasal administration according to claims 7, wherein said filamentous bacteriophage is selected from the group consisting of a fd bacteriophage, M13 bacteriophage and f1 bacteriophage.

9. Filamentous bacteriophage for use in the treatment of multiple sclerosis by intranasal administration according to claims 7-8, wherein said filamentous bacteriophage is a fd bacteriophage.

10. Filamentous bacteriophage for use in the treatment of multiple sclerosis by intranasal administration according to any of the claims 7-9, wherein said filamentous bacteriophage comprises 150 copies of said MOG antigen.

11. Filamentous bacteriophage for use in the treatment of multiple sclerosis by intranasal administration according to any of the claims 7-10, wherein said filamentous bacteriophage comprises 3000 copies of said MOG antigen.

## Patentansprüche

1. Filamentöser Bacteriophage, der ein MOG-Antigen, umfassend Aminosäuren 37-44 von SEQ ID NO:18, fusioniert an ein Haupthüllprotein VIII an einer Oberfläche desselben, präsentiert.

2. Filamentöser Bacteriophage gemäß Anspruch 1, wobei der filamentöse Bacteriophage aus der Gruppe, bestehend aus einem fd-Bacteriophagen, M13-Bacteriophagen und f1-Bacteriophagen, ausgewählt ist.

3. Filamentöser Bacteriophage gemäß den Ansprüchen 1-2, wobei der filamentöse Bacteriophage ein fd-Bacteriophage ist.

4. Filamentöser Bacteriophage gemäß einem der Ansprüche 1-3, wobei der filamentöse Bacteriophage 150 Kopien des MOG-Antigens umfasst.

5. Filamentöser Bacteriophage gemäß einem der Ansprüche 1-4, wobei der filamentöse Bacteriophage 3000 Kopien des MOG-Antigens umfasst.

6. Pharmazeutische Zusammensetzung zu nasalen Verabreichung, die als aktives Ingrediens den filamentösen Bacteriophagen gemäß einem der Ansprüche 1-5 und einen pharmazeutisch annehmbaren Träger umfasst.

7. Filamentöser Bacteriophage, der ein MOG-Antigen, umfassend Aminosäuren 37-44 von SEQ ID NO:18, fusioniert an ein Haupthüllprotein VIII an einer Oberfläche davon, präsentiert, zur Verwendung bei der Behandlung von Multipler Sklerose durch intranasale Verabreichung.

8. Filamentöser Bacteriophage zur Verwendung in der Behandlung von Multipler Sklerose durch intranasale Verabreichung gemäß Anspruch 7, wobei der filamentöse Bacteriophage aus der Gruppe, bestehend aus einem fd-Bacteriophagen, M13-Bacteriophagen und f1-Bacterophagen, ausgewählt ist.

9. Filamentöser Bacteriophage zur Verwendung in der Behandlung von Multipler Sklerose durch intranasale Verabreichung gemäß den Ansprüchen 7-8, wobei der filamentöse Bacteriophage ein fd-Bacteriophage ist.

10. Filamentöser Bacteriophage zur Verwendung in der Behandlung von Multipler Sklerose durch intranasale Verabreichung gemäß einem der Ansprüche 7-9, wobei der filamentöse Bacteriophage 150 Kopien des MOG-Antigens umfasst.

11. Filamentöser Bacteriophage zur Verwendung in der Behandlung von Multipler Sklerose durch intranasale Verabreichung gemäß einem der Ansprüche 7-10, wobei der filamentöse Bacteriophage 3000 Kopien des MOG-Antigens umfasst.

## Revendications

1. Bactériophage filamenteux présentant un antigène MOG comprenant les acides aminés 37 à 44 de SEQ ID N° : 18 fusionné à une protéine d'enveloppe VIII majeure sur une surface de celui-ci.

2. Bactériophage filamenteux selon la revendication 1, ledit bactériophage filamenteux étant choisi dans le groupe consistant en un bactériophage fd, un bactériophage M13 et un bactériophage f1.

3. Bactériophage filamenteux selon les revendications 1 à 2, ledit bactériophage filamenteux étant un bactériophage fd.

4. Bactériophage filamenteux selon l'une quelconque des revendications 1 à 3, ledit bactériophage filamenteux comprenant 150 copies dudit antigène MOG.

5. Bactériophage filamenteux selon l'une quelconque des revendications 1 à 4, ledit bactériophage filamenteux comprenant 3000 copies dudit antigène MOG.

6. Composition pharmaceutique pour l'administration nasale comprenant, comme ingrédient actif, le bactériophage filamenteux selon l'une quelconque des revendications 1 à 5 et un véhicule pharmaceutiquement acceptable.

7. Bactériophage filamenteux présentant un antigène MOG comprenant les acides aminés 37 à 44 de SEQ ID N° : 18 fusionné à une protéine d'enveloppe VIII majeure sur une surface de celui-ci pour l'utilisation dans le traitement de la sclérose en plaques par administration intranasale.

8. Bactériophage filamenteux pour l'utilisation dans le traitement de la sclérose en plaques par administration intranasale selon la revendication 7, ledit bactériophage filamenteux étant choisi dans le groupe consistant en un bactériophage fd, un bactériophage M13 et un bactériophage f1.

9. Bactériophage filamenteux pour l'utilisation dans le traitement de la sclérose en plaques par administration intranasale selon les revendications 7 à 8, ledit bactériophage filamenteux étant un bactériophage fd.

10. Bactériophage filamenteux pour l'utilisation dans le traitement de la sclérose en plaques par administration intranasale selon l'une quelconque des revendications 7 à 9, ledit bactériophage filamenteux comprenant 150 copies dudit antigène MOG.

11. Bactériophage filamenteux pour l'utilisation dans le traitement de la sclérose en plaques par administration intranasale selon l'une quelconque des revendications 7 à 10, ledit bactériophage filamenteux comprenant 3000 copies dudit antigène MOG.

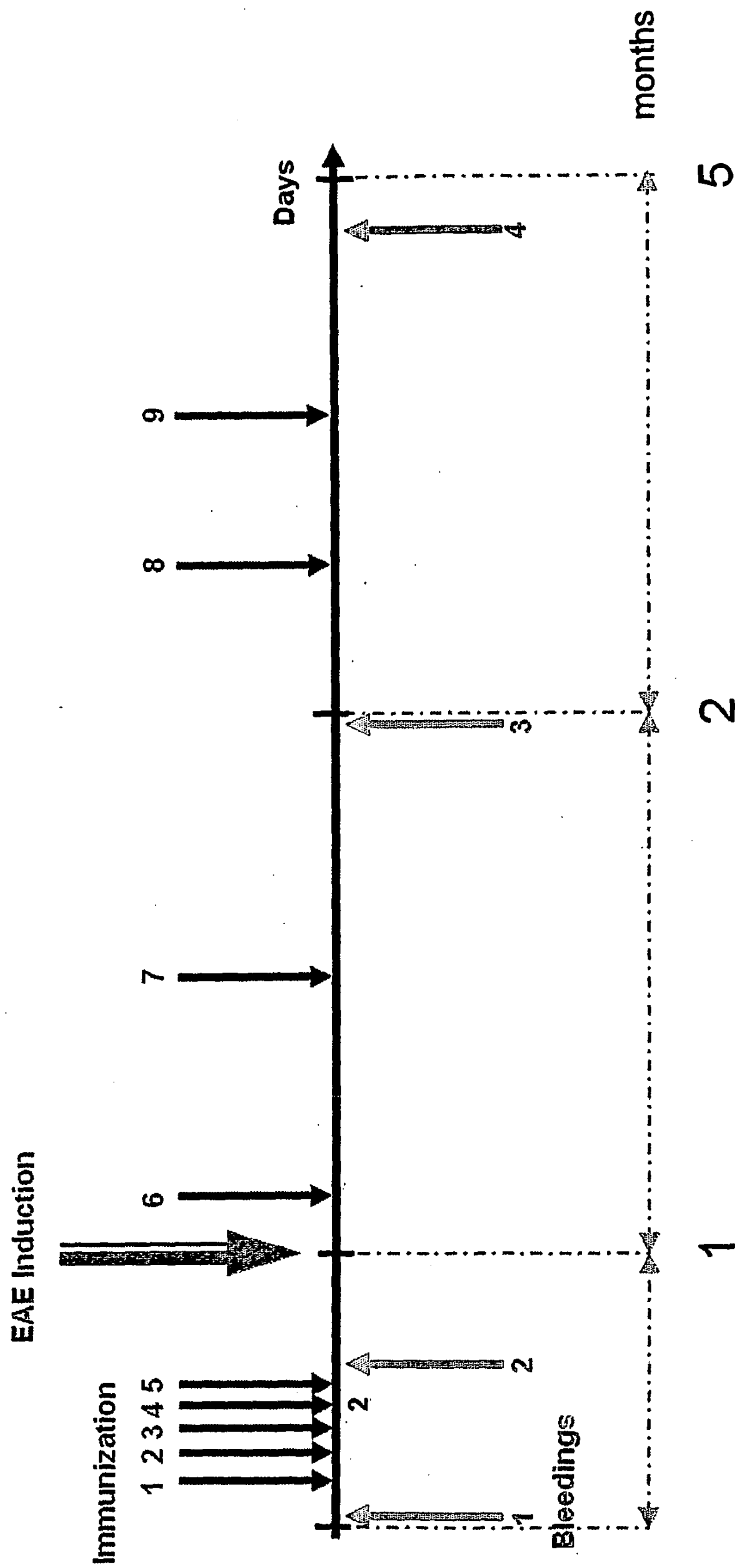
Immunization
1 2 3 4 5
EAE Induction
6
7
8
9
Days
Bleedings
1
2
2
3
4
1
2
5
months

Fig. 1

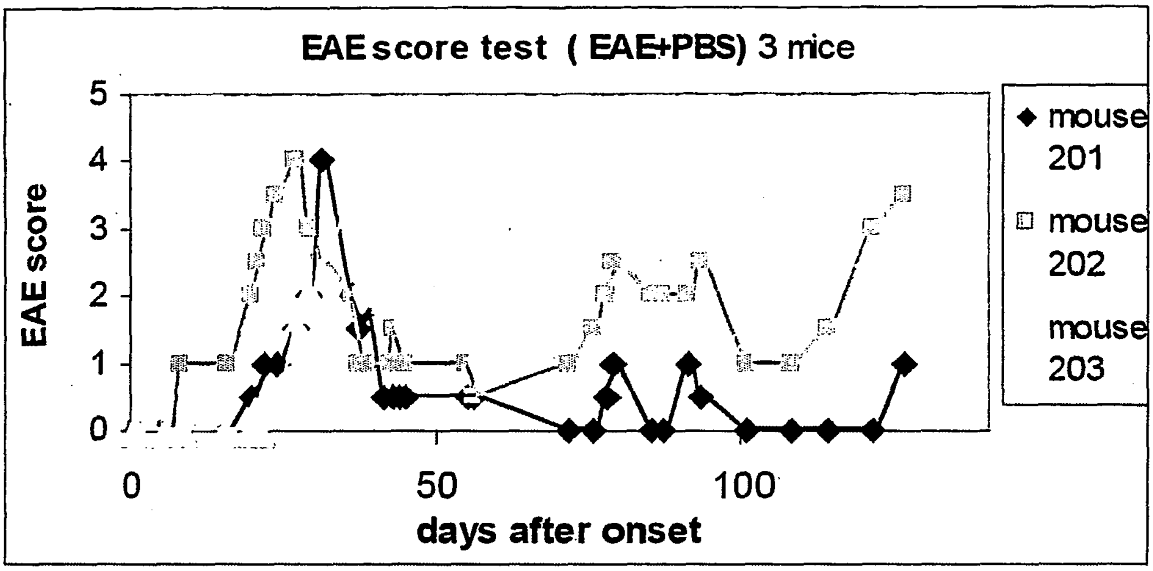
EAE score test ( EAE+PBS) 3 mice
EAE score
days after onset
0
50
100
0
1
2
3
4
5
mouse 201
mouse 202
mouse 203

Fig. 2a

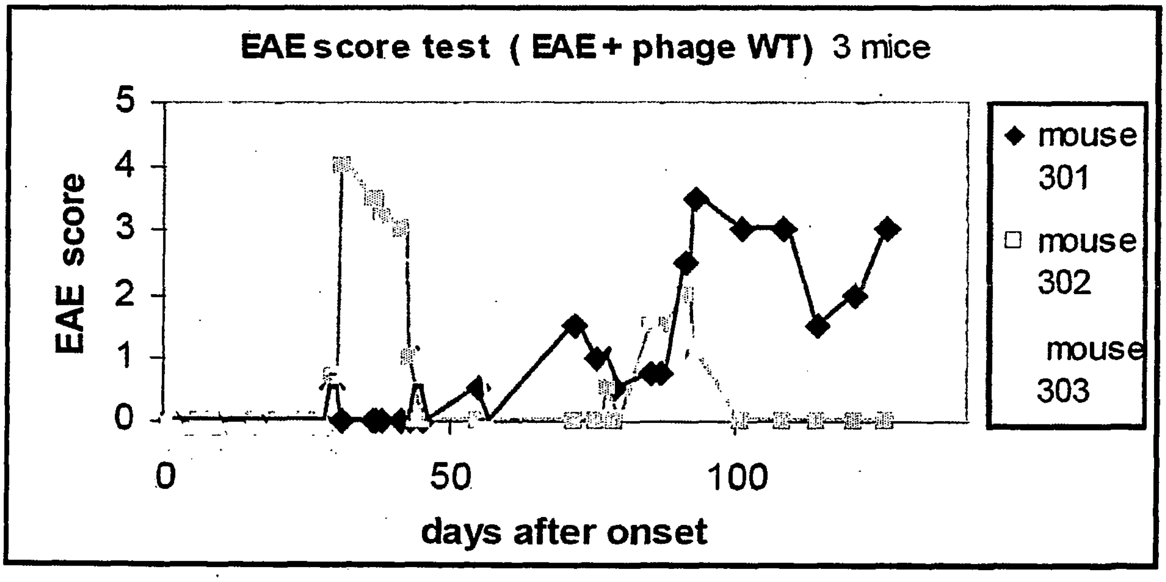
EAE score test ( EAE + phage WT) 3 mice
EAE score
days after onset
0
50
100
0
1
2
3
4
5
mouse 301
mouse 302
mouse 303

Fig. 2b

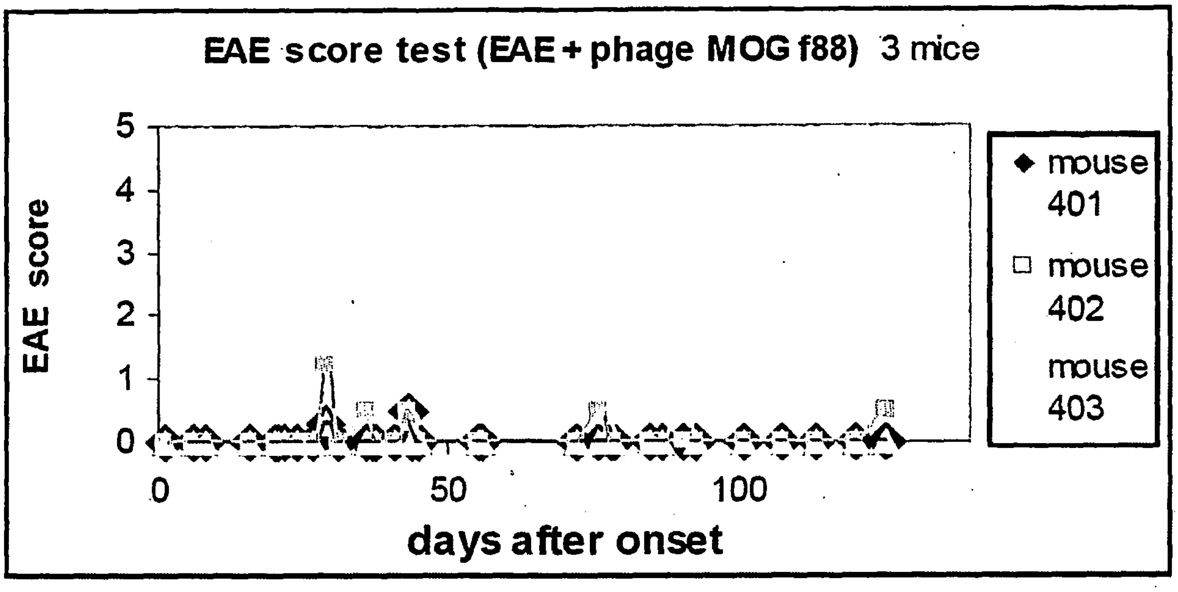
EAE score test (EAE + phage MOG f88) 3 mice
EAE score
0
1
2
3
4
5
0
50
100
days after onset
mouse 401
mouse 402
mouse 403

Fig. 2c

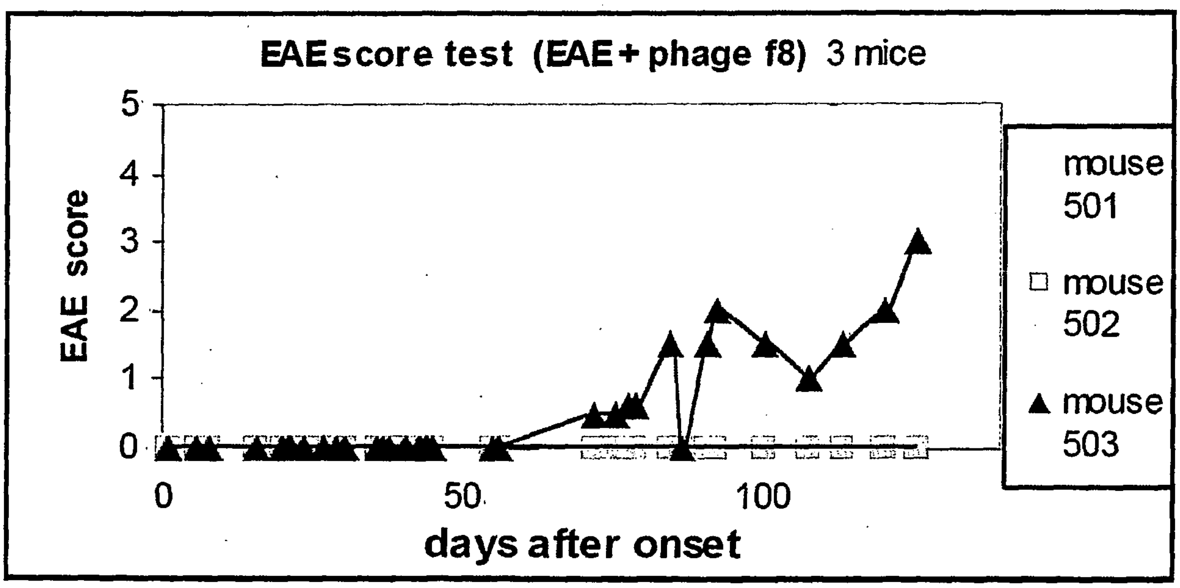
EAE score test (EAE + phage f8) 3 mice
EAE score
0
1
2
3
4
5
0
50
100
days after onset
mouse 501
mouse 502
mouse 503

Fig. 2d

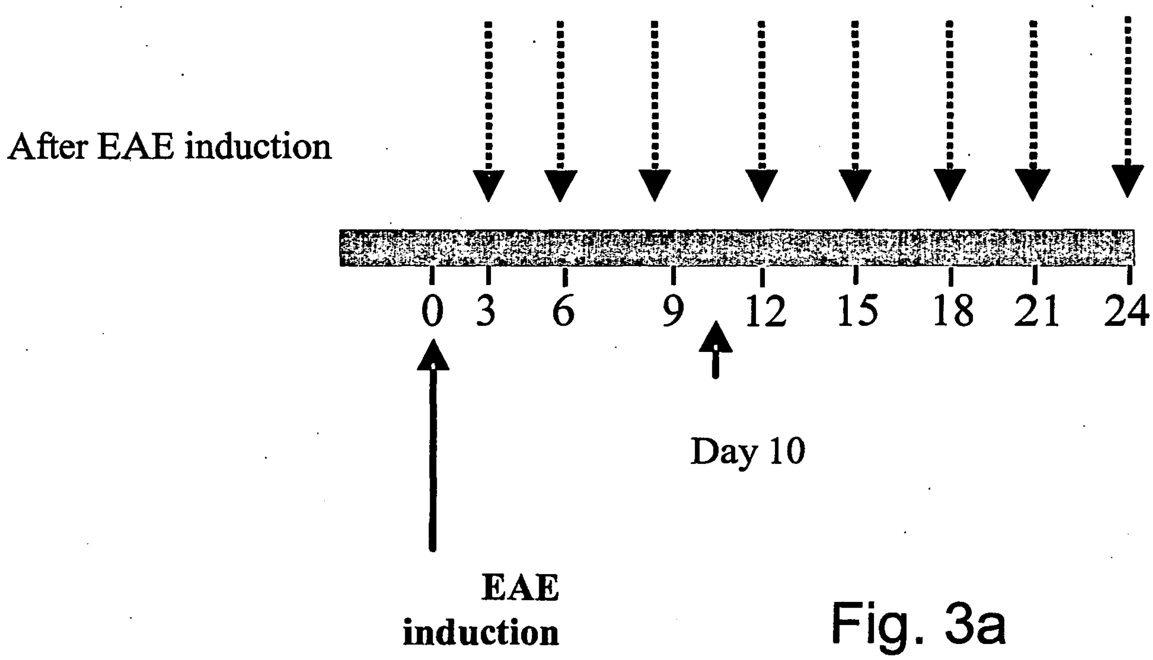
After EAE induction
0
3
6
9
12
15
18
21
24
Day 10
EAE induction

Fig. 3a

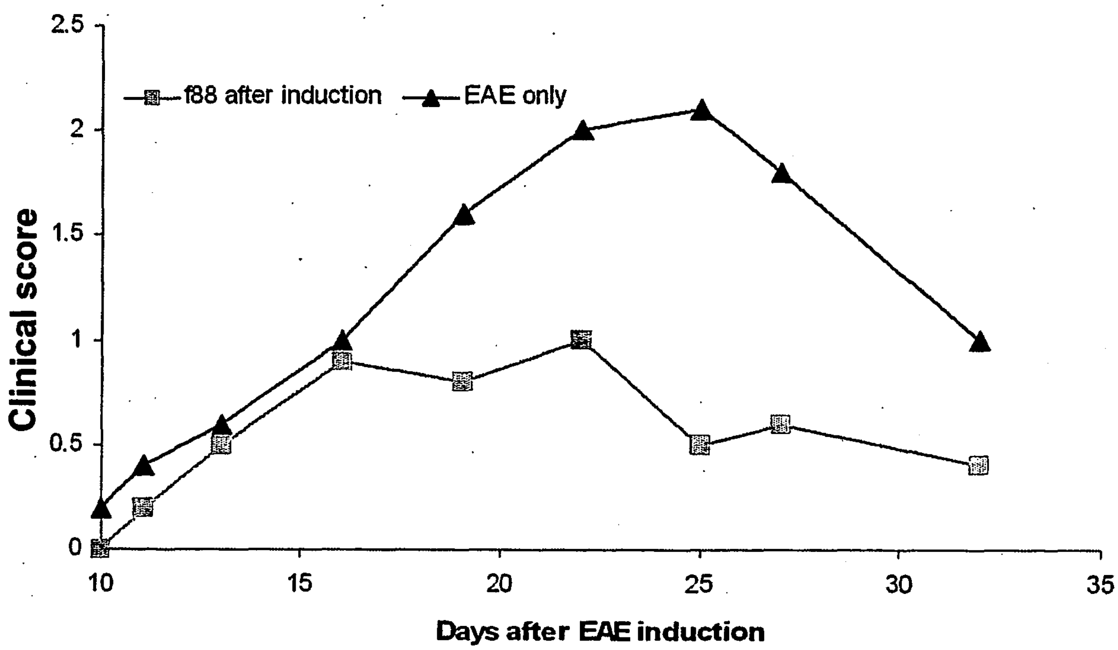
f88 after induction
EAE only
Clinical score
Days after EAE induction
2.5
2
1.5
1
0.5
0
10
15
20
25
30
35

Fig. 3b

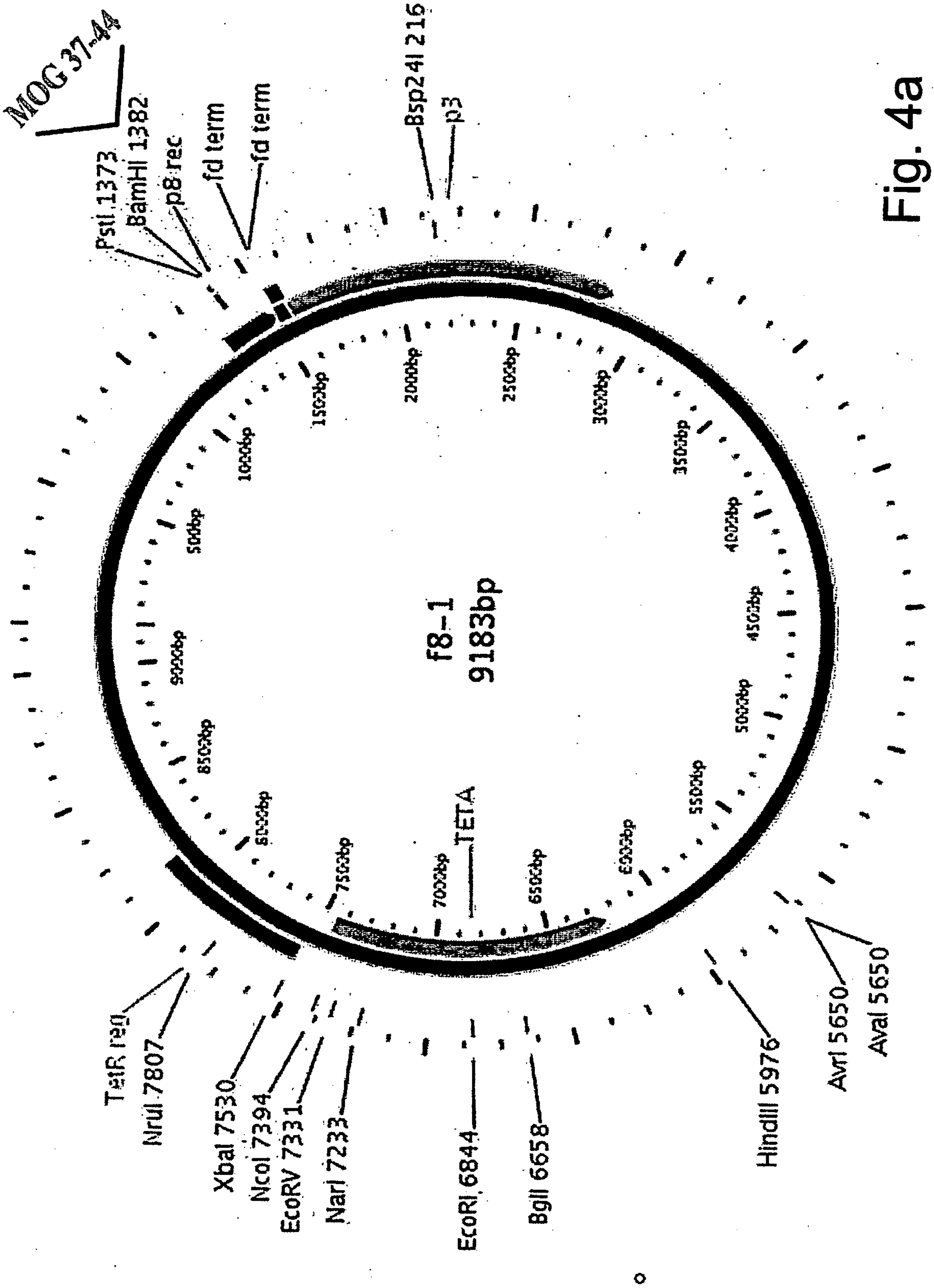
MOG 37-44
PstI 1373
BamHI 1382
p8 rec
fd term
fd term
Bsp24I 216
p3
f8-1
9183bp
TETA
TetR reg
NruI 7807
XbaI 7530
NcoI 7394
EcoRV 7331
NarI 7233
EcoRI 6844
BglI 6658
HindIII 5976
AvrI 5650
AvaI 5650

Fig. 4a

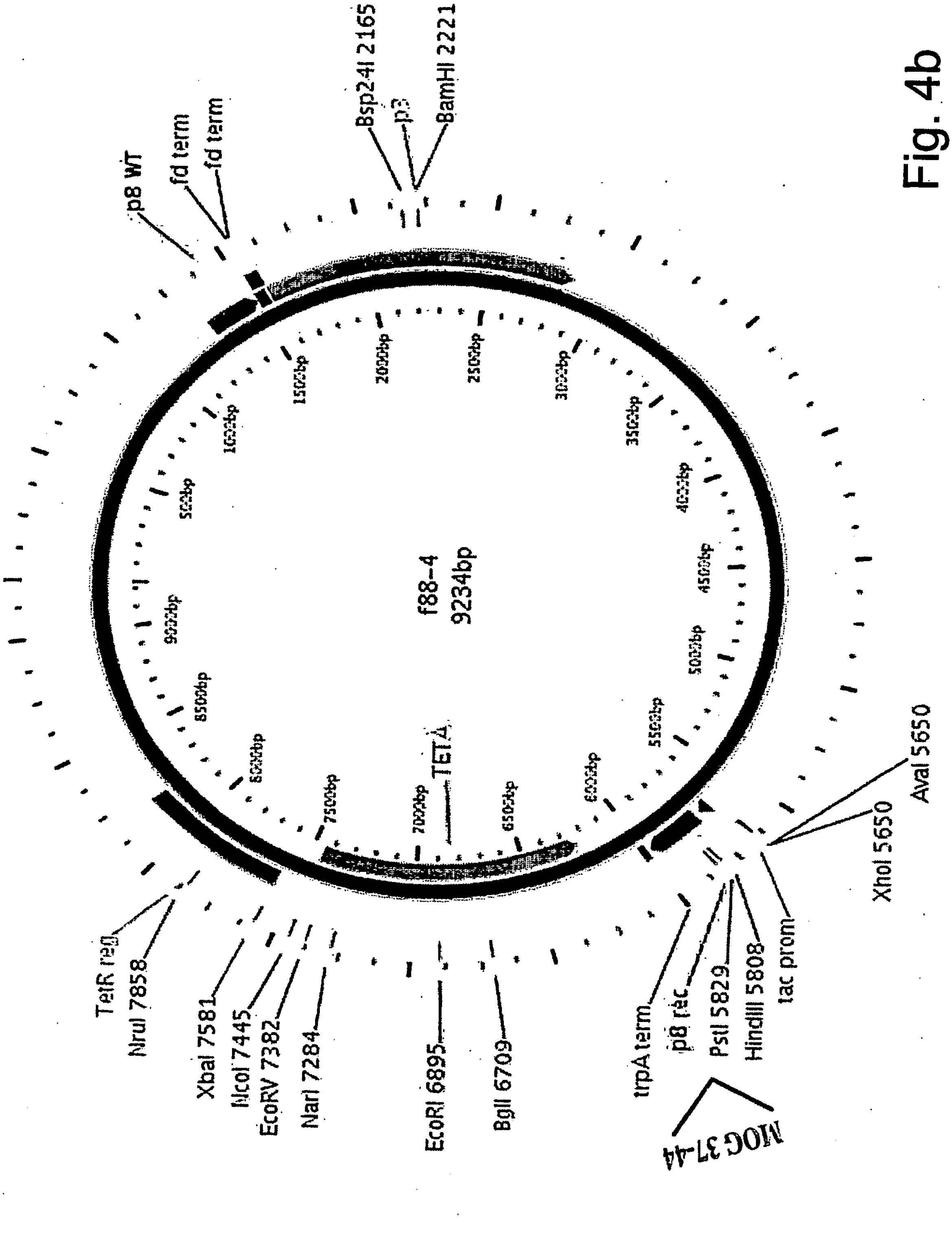
f88-4
9234bp
p8 WT
fd term
fd term
Bsp24I 2165
p3
BamHI 2221
AvaI 5650
XhoI 5650
tac prom
HindIII 5808
PstI 5829
p8 rec
trpA term
MOG 37-44
BglI 6709
EcoRI 6895
NarI 7284
EcoRV 7382
NcoI 7445
XbaI 7581
NruI 7858
TetR reg
TET A
500bp
1000bp
1500bp
2000bp
2500bp
3000bp
3500bp
4000bp
4500bp
5000bp
5500bp
6000bp
6500bp
7000bp
7500bp
8000bp
8500bp
9000bp

Fig. 4b

```
          1        10        20        30        40        50        60        70        80
          |--------+---------+---------+---------+---------+---------+---------+---------+------
       f8       ACCCTCGTTCCGATGCTGTCTTTCGCTGCAGAGGTGGGCTGGTATCGCAGTCCGTTTGAGGATCCCGCAAAAG--CGGC( SEQ ID NO: 22
      f88 GTTGCGACTCTTGTTCCTATGCTAAGCTT---TGCCGAGGTGGGCTGGTATCGCAGTCCGTTTATTGCAGAAGGTGATGACCCGG( SEQ ID NO: 17
      mog                                        GTGGGCTGGTATCGCAGTCCGTTT                          SEQ ID NO: 12
Consensus ......ac.ct.gttcc.atgct....tt...tgc.GAGGTGGGCTGGTATCGCAGTCCGTTT...g.....g...a.g..c.g.(
                                             V  G  W  Y  R  S  P  F                           SEQ ID NO: 10
```

Fig. 4c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5645820 A, to Hafler DA. and Weiner HL.
- US 6703015 B, Solomon B. and Frenkel D.
- US 5399347 A
- US 5720955 A
- US 5733542 A
- US 5843445 A
- US 5856446 A
- US 6019975 A
- US 5643868 A
- US 5763396 A
- US 5858968 A
- US 6645504 B
- US 6703361 B
- US 5961977 A
- US 5849298 A
- US 5858364 A
- US 5858980 A
- US 5869093 A
- US 6077509 A
- US 6812205 B
- US 5935577 A
- US 5397771 A
- US 4690683 A, Weiner
- US 6790447 B, Wildner
- EP 0886471 A1
- WO 01821951 A, Haas
- US 5843449 A, Boots
- US 20040029786 A, Das
- US 4704295 A
- US 4556552 A
- US 4309404 A
- US 4309406 A
- US 5405619 A
- WO 8502092 A
- US 5416071 A
- US 5371109 A
- US 5356635 A
- US 5236704 A
- US 5151272 A
- US 4985253 A
- US 4895724 A
- US 4675189 A
- EP 516141 A
- US 4226848 A
- US 4713243 A
- US 4940587 A
- EP 205282 A
- US 5284660 A
- US 5141750 A
- US 5110597 A
- US 4917895 A
- US 4837027 A
- US 3993073 A
- US 3948262 A
- US 3944064 A
- US 3699963 A
- WO 9511011 A
- WO 9515191 A
- EP 259013 A
- EP 354742 A
- US 4624251 A
- US 3703173 A
- US 3561444 A
- US 4635627 A
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A


### Non-patent literature cited in the description


- **MIYAMOTO et al.** *Journal of Immunology,* 2005, vol. 175 (11), 7341-7347

- **JOHNS, T.G. ; C.C. BERNARD.** The structure and function of myelin oligodendrocyte glycoprotein. *J. Neurochem.,* 1999, vol. 72 (1), 1-9
- **SHAO H ; HUANG Z ; SUN SL ; KAPLAN HJ ; SUN D.** Myelin/oligodendrocyte glycoprotein-specific T-cells induce severe optic neuritis in the C57BL/6 mouse. *Invest Ophthalmol. Vis. Sci.,* 2004, vol. 45, 4060-5
- **HOLZ A et al.** *J. Immunol.,* 2000, vol. 164 (2), 1103-9
- **JANICE S. et al.** Type 1 Diabetes and Multiple Sclerosis: Together at last. *Diabetes Care,* 2003, vol. 26, 3192-3193
- **SMART J D et al.** *J Pharm Pharmacol,* 1984, vol. 36, 295-9
- **SALA et al.** *Proceed Intern Symp Control Rel Bioact Mater,* 1989, vol. 16, 420-1
- **HUNTER et al.** *International Journal of Pharmaceutics,* 1983, vol. 17, 59-64
- Bioadhesion-Possibilities and Future Trends, Kellaway. *Course No. 470,* 22 May 1989
- **NEWMAN S P.** Aerosols and the Lung. Butterworths, 1984, 197-224
- **SHAO H ; HUANG Z ; SUN SL ; KAPLAN HJ ; SUN D.** Myelin/oligodendrocyte glycoprotein-specific T-cells induce severe optic neuritis in the C57BL/6 mouse. *Invest Ophthalmol Vis Sci.,* 2004, vol. 45, 4060-5
- **FINGL et al.** The Pharmacological Basis of Therapeutics. 1975, 1
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- A Practical Guide to Molecular Cloning. Perbal, B, 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **SMITH GP ; SCOTT JK.** Libraries of peptides and proteins displayed on filamentous phage. *Methods Enzymol,* 1993, vol. 217, 228-257
- **SMITH GP.** Surface presentation of protein epitopes using bacteriophage expression systems. *Curr Opin Biotechnol,* 1991, vol. 2, 668-673
- **AMOR, S. ; N. GROOME ; C. LININGTON et al.** Identification of epitopes of myelin oligodendrocyte glycoprotein for the induction of experimental allergic encephalomyelitis in SJL and Biozzi AB/H mice. *J Immunol,* 1994, vol. 153 (10), 4349-4356
- **BAI, X.-F. ; F.-D. SHI ; B.-G. XIAO et al.** Nasal administration of myelin basic protein prevents relapsing experimental autoimmune encephalomyelitis in DA rats by activating regulatory cells expressing IL-4 and TGF-[beta] mRNA. *Journal ofNeuroimmunology,* 1997, vol. 80 (1-2), 65-75
- **FRIEDMAN, A. ; H. L. WEINER.** Induction of Anergy or Active Suppression Following Oral Tolerance is Determined by Antigen Dosage. *PNAS,* 1994, vol. 91 (14), 6688-6692
- **KERLERO DE ROSBO, N. ; M. HOFFMAN ; I. MENDEL et al.** Predominance of the autoimmune response to myelin oligodendrocyte glycoprotein (MOG) in multiple sclerosis: reactivity to the extracellular domain of MOG is directed against three main regions. *Eur J Immunol,* 1997, vol. 27 (11), 3059-69
- **PHAM-DINH, D. ; M. MATTEI ; J. NUSSBAUM et al.** Myelin/Oligodendrocyte Glycoprotein is a Member of a Subset of the Immunoglobulin Superfamily Encoded within the Major Histocompatibility Complex. *PNAS,* 1993, vol. 90 (17), 7990-7994
- **PARKMAN.** Graft-versus-host Disease. *Ann. Rev. Med.,* 1991, vol. 42, 189-197
- **SAO H. et al.** *Invest. Ophthalmol. Vis. Sci.,* 2004, vol. 45, 4060-4065
- **SUN D. et al.** *The Journal of Immunology,* 2001, vol. 166, 7579-7587

SEQUENCE LISTING

<110> Solomon, Beka
Zabavnik, Natalia
Koppel, Rela
Rakover Idan

<120> VIRAL DISPLAY VEHICLES FOR TREATING MULTIPLE SCLEROSIS

<130> 33255

<160> 22

<170> PatentIn version 3.4

<210> 1
<211> 30
<212> DNA
<213> Artificial sequence

<220>
<223> Single strand DNA oligonucleotide

<400> 1
gaggtgggct ggtatcgcag tccgtttgag 30

<210> 2
<211> 38
<212> DNA
<213> Artificial sequence

<220>
<223> Single strand DNA oligonucleotide

<400> 2
gatcctcaaa cggactgcga taccagccca cctctgca 38

<210> 3
<211> 42
<212> DNA
<213> Artificial sequence

<220>
<223> Single strand DNA oligonucleotide

<400> 3
agctttgccg aggtgggctg gtatcgcagt ccgtttattg ca 42

<210> 4
<211> 34
<212> DNA
<213> Artificial sequence

<220> Single strand DNA oligonucleotide
<223> Single strand DNA oligonucleotide

<400> 4
ataaacggac tgcgatacca gcccacctcg gcaa 34

<210> 5
<211> 19
<212> DNA
<213> Artificial sequence

<220>
<223> Single strand DNA oligonucleotide

<400> 5
cagcttgctt tcgagatga 19

<210> 6

<211> 19
<212> DNA
<213> Artificial sequence

<220>
<223> Single strand DNA oligonucleotide

<400> 6
agtagcagaa gcctgaaga 19

<210> 7
<211> 218
<212> PRT
<213> Mus musculus

<400> 7

```
Gly Gln Phe Arg Val Ile Gly Pro Gly Tyr Pro Ile Arg Ala Leu Val
1               5                   10                  15

Gly Asp Glu Ala Glu Leu Pro Cys Arg Ile Ser Pro Gly Lys Asn Ala
            20                  25                  30

Thr Gly Met Glu Val Gly Trp Tyr Arg Ser Pro Phe Ser Arg Val Val
        35                  40                  45

His Leu Tyr Arg Asn Gly Lys Asp Gln Asp Ala Glu Gln Ala Pro Glu
    50                  55                  60

Tyr Arg Glu Arg Thr Glu Leu Leu Lys Glu Thr Ile Ser Glu Gly Lys
65                  70                  75                  80

Val Thr Leu Arg Ile Gln Asn Val Arg Phe Ser Asp Glu Gly Gly Tyr
                85                  90                  95

Thr Cys Phe Phe Arg Asp His Ser Tyr Gln Glu Glu Ala Ala Met Glu
            100                 105                 110

Leu Lys Val Glu Asp Pro Phe Tyr Trp Val Asn Pro Gly Val Leu Thr
        115                 120                 125

Leu Ile Ala Leu Val Pro Thr Ile Leu Leu Gln Val Pro Val Gly Leu
    130                 135                 140

Val Phe Leu Phe Leu Gln His Arg Leu Arg Gly Lys Leu Arg Ala Glu
145                 150                 155                 160

Val Glu Asn Leu His Arg Thr Phe Asp Pro His Phe Leu Arg Val Pro
                165                 170                 175

Cys Trp Lys Ile Thr Leu Phe Val Ile Val Pro Val Leu Gly Pro Leu
            180                 185                 190

Val Ala Leu Ile Ile Cys Tyr Asn Trp Leu His Arg Arg Leu Ala Gly
        195                 200                 205

Gln Phe Leu Glu Glu Leu Arg Asn Pro Phe
    210                 215
```

<210> 8
<211> 304
<212> PRT
<213> Homo sapiens

<400> 8

```
Met Gly Asn His Ala Gly Lys Arg Glu Leu Asn Ala Glu Lys Ala Ser
1               5                   10                  15

Thr Asn Ser Glu Thr Asn Arg Gly Glu Ser Glu Lys Lys Arg Asn Leu
            20                  25                  30

Gly Glu Leu Ser Arg Thr Thr Ser Glu Asp Asn Glu Val Phe Gly Glu
        35                  40                  45

Ala Asp Ala Asn Gln Asn Asn Gly Thr Ser Ser Gln Asp Thr Ala Val
    50                  55                  60

Thr Asp Ser Lys Arg Thr Ala Asp Pro Lys Asn Ala Trp Gln Asp Ala
65                  70                  75                  80

His Pro Ala Asp Pro Gly Ser Arg Pro His Leu Ile Arg Leu Phe Ser
                85                  90                  95

Arg Asp Ala Pro Gly Arg Glu Asp Asn Thr Phe Lys Asp Arg Pro Ser
            100                 105                 110

Glu Ser Asp Glu Leu Gln Thr Ile Gln Glu Asp Ser Ala Ala Thr Ser
        115                 120                 125

Glu Ser Leu Asp Val Met Ala Ser Gln Lys Arg Pro Ser Gln Arg His
    130                 135                 140

Gly Ser Lys Tyr Leu Ala Thr Ala Ser Thr Met Asp His Ala Arg His
145                 150                 155                 160

Gly Phe Leu Pro Arg His Arg Asp Thr Gly Ile Leu Asp Ser Ile Gly
                165                 170                 175

Arg Phe Phe Gly Gly Asp Arg Gly Ala Pro Lys Arg Gly Ser Gly Lys
            180                 185                 190

Asp Ser His His Pro Ala Arg Thr Ala His Tyr Gly Ser Leu Pro Gln
        195                 200                 205

Lys Ser His Gly Arg Thr Gln Asp Glu Asn Pro Val Val His Phe Phe
    210                 215                 220

Lys Asn Ile Val Thr Pro Arg Thr Pro Pro Pro Ser Gln Gly Lys Gly
225                 230                 235                 240

Arg Gly Leu Ser Leu Ser Arg Phe Ser Trp Gly Ala Glu Gly Gln Arg
                245                 250                 255

Pro Gly Phe Gly Tyr Gly Gly Arg Ala Ser Asp Tyr Lys Ser Ala His
            260                 265                 270

Lys Gly Phe Lys Gly Val Asp Ala Gln Gly Thr Leu Ser Lys Ile Phe
        275                 280                 285

Lys Leu Gly Gly Arg Asp Ser Arg Ser Gly Ser Pro Met Ala Arg Arg
    290                 295                 300
```

<210> 9
<211> 21
<212> PRT
<213> Artificial sequence

<220>
<223> MOG 35-55 epitope polypeptide

<400> 9

```
Met Glu Val Gly Trp Tyr Arg Ser Pro Phe Ser Arg Val Val His Leu
1               5                   10                  15

Tyr Arg Asn Gly Lys
            20
```

<210> 10
<211> 8
<212> PRT
<213> Artificial sequence

<220>
<223> Mouse MOG 37-44 polypeptide

<400> 10

```
Val Gly Trp Tyr Arg Ser Pro Phe
1               5
```

<210> 11
<211> 9183
<212> DNA
<213> Artificial sequence

<220>
<223> Filamentous phage display vector f8-1

<400> 11

```
aacgctacta ccattagtag aattgatgcc acctttttcag ctcgcgcccc aaatgaaaat     60
atagctaaac aggttattga ccatttgcga aatgtatcta atggtcaaac taaatctact     120
cgttcgcaga attgggaatc aactgttaca tggaatgaaa cttccagaca ccgtacttta     180
gttgcatatt taaaacatgt tgaactacag caccagattc agcaattaag ctctaagcca     240
tccgcaaaaa tgacctctta tcaaaaggag caattaaagg tactgtctaa tcctgacctg     300
ttggaatttg cttccggtct ggttcgcttt gaggctcgaa ttgaaacgcg atatttgaag     360
tctttcgggc ttcctcttaa tctttttgat gcaattcgct ttgcttctga ctataataga     420
cagggtaaag acctgatttt tgatttatgg tcattctcgt tttctgaact gtttaaagca     480
tttgaggggg attcaatgaa tatttatgac gattccgcag tattggacgc tatccagtct     540
aaacatttta caattacccc ctctggcaaa acttcctttg caaaagcctc tcgctatttt     600
ggtttctatc gtcgtctggt taatgagggt tatgatagtg ttgctcttac catgcctcgt     660
aattcctttt ggcgttatgt atctgcatta gttgagtgtg gtattcctaa atctcaattg     720
atgaatcttt ccacctgtaa taatgttgtt ccgttagttc gttttattaa cgtagatttt     780
```

tcctcccaac gtcctgactg gtataatgag ccagttctta aaatcgcata aggtaattca 840
aaatgattaa agttgaaatt aaaccgtctc aagcgcaatt tactacccgt tctggtgttt 900
ctcgtcaggg caagccttat tcactgaatg agcagctttg ttacgttgat ttgggtaatg 960
aatatccggt gcttgtcaag attactctcg acgaaggtca gccagcgtat gcgcctggtc 1020
tgtacaccgt gcatctgtcc tcgttcaaag ttggtcagtt cggttctctt atgattgacc 1080
gtctgcgcct cgttccggct aagtaacatg gagcaggtcg cggatttcga cacaatttat 1140
caggcgatga tacaaatctc cgttgtactt tgtttcgcgc ttggtataat cgctgggggt 1200
caaagatgag tgttttagtg tattctttcg cctctttcgt tttaggttgg tgccttcgta 1260
gtggcattac gtattttacc cgtttaatgg aaacttcctc atgaaaaagt ctttagtcct 1320
caaagcctcc gtagccgttg ctaccctcgt tccgatgctg tctttcgctg cagagggtga 1380
ggatcccgca aaagcggcct ttgactccct gcaagcctca gcgaccgaat atatcggtta 1440
tgcgtgggcg atggttgttg tcattgtcgg cgcaactatc ggtatcaagc tgtttaagaa 1500
attcacctcg aaagcaagct gataaaccga tacaattaaa ggctcctttt ggagcctttt 1560
tttttggaga ttttcaacgt gaaaaaatta ttattcgcaa ttcctttagt tgttcctttc 1620
tattctcact ccgctgaaac tgttgaaagt tgtttagcaa aacctcatac agaaaattca 1680
tttactaacg tctggaaaga cgacaaaact ttagatcgtt acgctaacta tgagggctgt 1740
ctgtggaatg ctacaggcgt tgtggtttgt actggtgacg aaactcagtg ttacggtaca 1800
tgggttccta ttgggcttgc tatccctgaa aatgagggtg gtggctctga gggtggcggt 1860
tctgagggtg gcggttctga gggtggcggt actaaacctc ctgagtacgg tgatacacct 1920
attccgggct atacttatat caaccctctc gacggcactt atccgcctgg tactgagcaa 1980
aaccccgcta atcctaatcc ttctcttgag gagtctcagc ctcttaatac tttcatgttt 2040
cagaataata ggttccgaaa taggcagggt gcattaactg tttatacggg cactgttact 2100
caaggcactg accccgttaa aacttattac cagtacactc ctgtatcatc aaaagccatg 2160
tatgacgctt actggaacgg taaattcaga gactgcgctt tccattctgg ctttaatgaa 2220
gatccattcg tttgtgaata tcaaggccaa tcgtctgacc tgcctcaacc tcctgtcaat 2280
gctggcggcg gctctggtgg tggttctggt ggcggctctg agggtggcgg ctctgagggt 2340
ggcggttctg agggtggcgg ctctgagggt ggcggttccg gtggcggctc cggttccggt 2400
gattttgatt atgaaaaaat ggcaaacgct aataaggggg ctatgaccga aaatgccgat 2460
gaaaacgcgc tacagtctga cgctaaaggc aaacttgatt ctgtcgctac tgattacggt 2520
gctgctatcg atggtttcat tggtgacgtt tccggccttg ctaatggtaa tggtgctact 2580
ggtgattttg ctggctctaa ttcccaaatg gctcaagtcg gtgacggtga taattcacct 2640
ttaatgaata atttccgtca atatttacct tctttgcctc agtcggttga atgtcgccct 2700
tatgtctttg gcgctggtaa accatatgaa ttttctattg attgtgacaa aataaactta 2760
ttccgtggtg tctttgcgtt tcttttatat gttgccacct ttatgtatgt attttcgacg 2820
tttgctaaca tactgcgtaa taaggagtct taatcatgcc agttcttttg ggtattccgt 2880
tattattgcg tttcctcggt ttccttctgg taactttgtt cggctatctg cttactttcc 2940
ttaaaaaggg cttcggtaag atagctattg ctatttcatt gtttcttgct cttattattg 3000

```
ggcttaactc aattcttgtg ggttatctct ctgatattag cgcacaatta ccctctgatt    3060
ttgttcaggg cgttcagtta attctcccgt ctaatgcgct tccctgtttt tatgttattc    3120
tctctgtaaa ggctgctatt ttcatttttg acgttaaaca aaaaatcgtt tcttatttgg    3180
attgggataa ataaatatgg ctgtttattt tgtaactggc aaattaggct ctggaaagac    3240
gctcgttagc gttggtaaga ttcaggataa aattgtagct gggtgcaaaa tagcaactaa    3300
tcttgattta aggcttcaaa acctcccgca agtcgggagg ttcgctaaaa cgcctcgcgt    3360
tcttagaata ccggataagc cttctatttc tgatttgctt gctattggtc gtggtaatga    3420
ttcctacgac gaaaataaaa acggtttgct tgttcttgat gaatgcggta cttggtttaa    3480
tacccgttca tggaatgaca aggaaagaca gccgattatt gattggtttc ttcatgctcg    3540
taaattggga tgggatatta tttttcttgt tcaggattta tctattgttg ataaacaggc    3600
gcgttctgca ttagctgaac acgttgttta ttgtcgccgt ctggacagaa ttactttacc    3660
ctttgtcggc actttatatt ctcttgttac tggctcaaaa atgcctctgc ctaaattaca    3720
tgttggtgtt gttaaatatg gtgattctca attaagccct actgttgagc gttggcttta    3780
tactggtaag aatttatata acgcatatga cactaaacag gctttttcca gtaattatga    3840
ttcaggtgtt tattcatatt taacccctta tttatcacac ggtcggtatt tcaaaccatt    3900
aaatttaggt cagaagatga aattaactaa aatatatttg aaaaagtttt ctcgcgttct    3960
ttgtcttgcg ataggatttg catcagcatt tacatatagt tatataaccc aacctaagcc    4020
ggaggttaaa aaggtagtct ctcagaccta tgattttgat aaattcacta ttgactcttc    4080
tcagcgtctt aatctaagct atcgctatgt tttcaaggat tctaagggaa aattaattaa    4140
tagcgacgat ttacagaagc aaggttattc catcacatat attgatttat gtactgtttc    4200
aattaaaaaa ggtaattcaa atgaaattgt taaatgtaat taattttgtt ttcttgatgt    4260
ttgtttcatc atcttctttt gctcaagtaa ttgaaatgaa taattcgcct ctgcgcgatt    4320
tcgtgacttg gtattcaaag caaacaggtg aatctgttat tgtctcacct gatgttaaag    4380
gtacagtgac tgtatattcc tctgacgtta agcctgaaaa tttacgcaat ttctttatct    4440
ctgttttacg tgctaataat tttgatatgg ttggctcaat tccttccata attcagaaat    4500
ataacccaaa tagtcaggat tatattgatg aattgccatc atctgatatt caggaatatg    4560
atgataattc cgctccttct ggtggtttct ttgttccgca aaatgataat gttactcaaa    4620
catttaaaat taataacgtt cgcgcaaagg atttaataag ggttgtagaa ttgtttgtta    4680
aatctaatac atctaaatcc tcaaatgtat tatctgttga tggttctaac ttattagtag    4740
ttagcgcccc taaagatatt ttagataacc ttccgcaatt tctttctact gttgatttgc    4800
caactgacca gatattgatt gaaggattaa ttttcgaggt tcagcaaggt gatgctttag    4860
atttttcctt tgctgctggc tctcagcgcg gcactgttgc tggtggtgtt aatactgacc    4920
gtctaacctc tgttttatct tctgcgggtg gttcgttcgg tatttttaac ggcgatgttt    4980
tagggctatc agttcgcgca ttaaagacta atagccattc aaaaatattg tctgtgcctc    5040
gtattcttac gctttcaggt cagaagggtt ctatttctgt tggccagaat gtccctttta    5100
ttactggtcg tgtaactggt gaatctgcca atgtaaataa tccatttcag acggttgagc    5160
gtcaaaatgt tggtatttct atgagtgttt ttcccgttgc aatggctggc ggtaatattg    5220
ttttagatat aaccagtaag gccgatagtt tgagttcttc tactcaggca agtgatgtta    5280
```

ttactaatca aagaagtatt gcgacaacgg ttaatttgcg tgatggtcag actcttttgc 5340

tcggtggcct cactgattac aaaaacactt ctcaagattc tggtgtgccg ttcctgtcta 5400

aaatcccttt aatcggcctc ctgtttagct cccgttctga ttctaacgag gaaagcacgt 5460

tgtacgtgct cgtcaaagca accatagtac gcgccctgta gcggcgcatt aagcgcggcg 5520

ggtgtggtgg ttacgcgcag cgtgaccgct acacttgcca gcgccctagc gcccgctcct 5580

ttcgctttct tcccttcctt tctcgccacg ttctccggct ttccccgtca agctctaaat 5640

cgggggatct cgggaaaagc gttggtgacc aaaggtgcct tttatcatca ctttaaaaat 5700

aaaaaacaat tactcagtgc ctgttataag cagcaattaa ttatgattga tgcctacatc 5760

acaacaaaaa ctgatttaac aaatggttgg tctgccttag aaagtatatt tgaacattat 5820

cttgattata ttattgataa taataaaaac cttatcccta tccaagaagt gatgcctatc 5880

attggttgga atgaacttga aaaaattagc cttgaataca ttactggtaa ggtaaacgcc 5940

attgtcagca aattgatcca agagaaccaa cttaaagctt atgatgatga tgtgcttaaa 6000

aacttactca atggctggtt tatgcatatc gcaatacatg cgaaaaacct aaaagagctt 6060

gccgataaaa aaggccaatt tattgctatt taccgcggct ttttattgag cttgaaagat 6120

aaataaaata gataggtttt atttgaagct aaatcttctt tatcgtaaaa aatgccctct 6180

tgggttatca agagggtcat tatatttcgc ggaataacat catttggtga cgaaataact 6240

aagcacttgt ctcctgttta ctcccctgag cttgaggggt taacatgaag gtcatcgata 6300

gcaggataat aatacagtaa aacgctaaac caataatcca aatccagcca tcccaaattg 6360

gtagtgaatg attataaata acagcaaaca gtaatgggcc aataacaccg gttgcattgg 6420

taaggctcac caataatccc tgtaaagcac cttgctgatg actctttgtt tggatagaca 6480

tcactccctg taatgcaggt aaagcgatcc caccaccagc caataaaatt aaaacaggga 6540

aaactaacca accttcagat ataaacgcta aaaaggcaaa tgcactacta tctgcaataa 6600

atccgagcag tactgccgtt ttttcgcccc atttagtggc tattcttcct gccacaaagg 6660

cttggaatac tgagtgtaaa agaccaagac ccgctaatga aaagccaacc atcatgctat 6720

tccatccaaa acgattttcg gtaaatagca cccacaccgt tgcgggaatt tggcctatca 6780

attgcgctga aaaataaata atcaacaaaa tgggcatcgt tttaaataaa gtgatgtata 6840

ccgaattcga ttgcgtctca acccctactt cggtatctgt attatcacgt gtatttttgg 6900

tttcacggaa ccaaaaacata accacaagga aagtgacaat atttagcaac gcagcgataa 6960

aaaagggact atgcggtgaa atctctcctg caaaaccacc aataataggc cccgctatta 7020

aaccaagccc aaaacttgcc cctaaccaac cgaaccactt cacgcgttga gaagctgagg 7080

tggtatcggc aatgaccgat gccgcgacag ccccagtagc tcctgtgatc cctgaaagca 7140

aacggcctaa atacagcatc caaagcgcac ttgaaaaagc cagcaataag taatccagcg 7200

atgcgcctat taatgacaac aacagcactg ggcgccgacc aaatcggtca gacatttttc 7260

caagccaagg agcaaagata acctgcatta acgcataaag tgcaagcaat acgccaaagt 7320

ggttagcgat atcttccgaa gcaataaatt cacgtaataa cgttggcaag actggcatga 7380

taaggccaat ccccatggca tcgagtaacg taattaccaa tgcgatcttt gtcgaactat 7440

tcatttcact ttctctatc actgataggg agtggtaaaa taactctatc aatgatagag 7500

```
tgtcaacaaa aattaggaat taatgatgtc tagattagat aaaagtaaag tgattaacag   7560
cgcattagag ctgcttaatg aggtcggaat cgaaggttta acaacccgta aactcgccca   7620
gaagctaggt gtagagcagc ctacattgta ttggcatgta aaaaataagc gggctttgct   7680
cgacgcctta gccattgaga tgttagatag gcaccatact cacttttgcc ctttagaagg   7740
ggaaagctgg caagattttt tacgtaataa cgctaaaagt tttagatgtg ctttactaag   7800
tcatcgcgat ggagcaaaag tacatttagg tacacggcct acagaaaaac agtatgaaac   7860
tctcgaaaat caattagcct ttttatgcca acaaggtttt tcactagaga atgcattata   7920
tgcactcagc gctgtggggc attttacttt aggttgcgta ttggaagatc aagagcatca   7980
agtcgctaaa gaagaaaggg aaacacctac tactgatagt atgccgccat tattacgaca   8040
agctatcgaa ttatttgatc accaaggtgc agagccagcc ttcttattcg gccttgaatt   8100
gatcatatgc ggattagaaa aacaacttaa atgtgaaagt gggtcttaaa agcagcataa   8160
ccttttttccg tgatggtaac ttcacggtaa ccaagatgtc gagttaacca ccctttagat   8220
tcataaagcg aaaataatgc ggctccaacg tacccaccta aatggaaacg gcgttcactc   8280
caatctaaac acgcacaaca gattttacgt gaatgtttgg aaggaacgtc aattcccatt   8340
tcatgaaaat attgaatacc acttaatgtg atcattgaac cattttcagt gatccattgc   8400
tgttgacaaa gggaatcata gatcccttta gggttccgat ttagtgcttt acggcacctc   8460
gacctccaaa aacttgattt gggtgatggt tcacgtagtg ggccatcgcc ctgatagacg   8520
gtttttcgcc ctttgacgtt ggagtccacg ttctttaata gtggactctt gttccaaact   8580
ggaacaacac tcacaactaa ctcggcctat tcttttgatt tataaggatt tttgtcattt   8640
tctgcttact ggttaaaaaa taagctgatt taacaaatat ttaacgcgaa atttaacaaa   8700
acattaacgt ttacaattta aatatttgct tatacaatca tcctgttttt ggggcttttc   8760
tgattatcaa tcggggtaca tatgattgac atgctagttt tacgattacc gttcatcgat   8820
tctcttgttt gctccagact ttcaggtaat gacctgatag cctttgtaga cctctcaaaa   8880
atagctaccc tctccggcat gaatttatca gctagaacgg ttgaatatca tattgacggt   8940
gatttgactg tctccggcct ttctcacccg tttgaatctt tgcctactca ttactccggc   9000
attgcattta aaatatatga gggttctaaa aatttttatc cctgcgttga aattaaggct   9060
tcaccagcaa aagtattaca gggtcataat gtttttggta caaccgattt agctttatgc   9120
tctgaggctt tattgcttaa ttttgctaac tctctgcctt gcttgtacga tttattggat   9180
gtt                                                                 9183
```

<210> 12
<211> 24
<212> DNA
<213> Artificial sequence

<220>
<223> Mouse MOG 37-44 peptide coding sequence

<400> 12
gtgggctggt atcgcagtcc gttt 24

<210> 13
<211> 81

<212> PRT
<213> Homo sapiens

<400> 13

```
Met Ser Gln Lys Pro Ala Lys Glu Gly Pro Arg Leu Ser Lys Asn Gln
1               5                   10                  15

Lys Tyr Ser Glu His Phe Ser Ile His Cys Cys Pro Pro Phe Thr Phe
            20                  25                  30

Leu Asn Ser Lys Lys Glu Ile Val Asp Arg Lys Tyr Ser Ile Cys Lys
        35                  40                  45

Ser Gly Cys Phe Tyr Gln Lys Lys Glu Glu Asp Trp Ile Cys Cys Ala
    50                  55                  60

Cys Gln Lys Thr Arg Leu Lys Arg Lys Ile Arg Pro Thr Pro Lys Lys
65                  70                  75                  80

Lys
```

<210> 14
<211> 207
<212> PRT
<213> Homo sapiens

<400> 14

```
Met Val Ala Thr Cys Leu Gln Val Val Gly Phe Val Thr Ser Phe Val
1               5                   10                  15

Gly Trp Ile Gly Val Ile Val Thr Thr Ser Thr Asn Asp Trp Val Val
            20                  25                  30

Thr Cys Gly Tyr Thr Ile Pro Thr Cys Arg Lys Leu Asp Glu Leu Gly
        35                  40                  45

Ser Lys Gly Leu Trp Ala Asp Cys Val Met Ala Thr Gly Leu Tyr His
    50                  55                  60

Cys Lys Pro Leu Val Asp Ile Leu Ile Leu Pro Gly Tyr Val Gln Ala
65                  70                  75                  80

Cys Arg Ala Leu Met Ile Ala Ala Ser Val Leu Gly Leu Pro Ala Ile
                85                  90                  95

Leu Leu Leu Leu Thr Val Leu Pro Cys Ile Arg Met Gly Gln Glu Pro
            100                 105                 110

Gly Val Ala Lys Tyr Arg Arg Ala Gln Leu Ala Gly Val Leu Leu Ile
        115                 120                 125

Leu Leu Ala Leu Cys Ala Leu Val Ala Thr Ile Trp Phe Pro Val Cys
    130                 135                 140

Ala His Arg Glu Thr Thr Ile Val Ser Phe Gly Tyr Ser Leu Tyr Ala
145                 150                 155                 160

Gly Trp Ile Gly Ala Val Leu Cys Leu Val Gly Gly Cys Val Ile Leu


                165                 170                 175

Cys Cys Ala Gly Asp Ala Gln Ala Phe Gly Glu Asn Arg Phe Tyr Tyr
            180                 185                 190

Thr Ala Gly Ser Ser Ser Pro Thr His Ala Lys Ser Ala His Val
        195                 200                 205
```

<210> 15
<211> 277
<212> PRT
<213> Homo sapiens

<400> 15

```
Met Gly Leu Leu Glu Cys Cys Ala Arg Cys Leu Val Gly Ala Pro Phe
1               5                   10                  15

Ala Ser Leu Val Ala Thr Gly Leu Cys Phe Phe Gly Val Ala Leu Phe
            20                  25                  30

Cys Gly Cys Gly His Glu Ala Leu Thr Gly Thr Glu Lys Leu Ile Glu
        35                  40                  45

Thr Tyr Phe Ser Lys Asn Tyr Gln Asp Tyr Glu Tyr Leu Ile Asn Val
    50                  55                  60

Ile His Ala Phe Gln Tyr Val Ile Tyr Gly Thr Ala Ser Phe Phe Phe
65                  70                  75                  80

Leu Tyr Gly Ala Leu Leu Leu Ala Glu Gly Phe Tyr Thr Thr Gly Ala
                85                  90                  95

Val Arg Gln Ile Phe Gly Asp Tyr Lys Thr Thr Ile Cys Gly Lys Gly
            100                 105                 110

Leu Ser Ala Thr Val Thr Gly Gly Gln Lys Gly Arg Gly Ser Arg Gly
        115                 120                 125

Gln His Gln Ala His Ser Leu Glu Arg Val Cys His Cys Leu Gly Lys
    130                 135                 140

Trp Leu Gly His Pro Asp Lys Phe Val Gly Ile Thr Tyr Ala Leu Thr
145                 150                 155                 160

Val Val Trp Leu Leu Val Phe Ala Cys Ser Ala Val Pro Val Tyr Ile
                165                 170                 175

Tyr Phe Asn Thr Trp Thr Thr Cys Gln Ser Ile Ala Phe Pro Ser Lys
            180                 185                 190

Thr Ser Ala Ser Ile Gly Ser Leu Cys Ala Asp Ala Arg Met Tyr Gly
        195                 200                 205

Val Leu Pro Trp Asn Ala Phe Pro Gly Lys Val Cys Gly Ser Asn Leu
    210                 215                 220

Leu Ser Ile Cys Lys Thr Ala Glu Phe Gln Met Thr Phe His Leu Phe

225                 230                 235                 240

Ile Ala Ala Phe Val Gly Ala Ala Ala Thr Leu Val Ser Leu Leu Thr
                245                 250                 255

Phe Met Ile Ala Ala Thr Tyr Asn Phe Ala Val Leu Lys Leu Met Gly
            260                 265                 270

Arg Gly Thr Lys Phe
        275
```

<210> 16
<211> 582
<212> PRT
<213> Homo sapiens

<400> 16

```
Met Ile Phe Leu Thr Ala Leu Pro Leu Phe Trp Ile Met Ile Ser Ala
1               5                   10                  15

Ser Arg Gly Gly His Trp Gly Ala Trp Met Pro Ser Ser Ile Ser Ala
            20                  25                  30

Phe Glu Gly Thr Cys Val Ser Ile Pro Cys Arg Phe Asp Phe Pro Asp
        35                  40                  45

Glu Leu Arg Pro Ala Val Val His Gly Val Trp Tyr Phe Asn Ser Pro
    50                  55                  60

Tyr Pro Lys Asn Tyr Pro Pro Val Val Phe Lys Ser Arg Thr Gln Val
65                  70                  75                  80

Val His Glu Ser Phe Gln Gly Arg Ser Arg Leu Leu Gly Asp Leu Gly
                85                  90                  95

Leu Arg Asn Cys Thr Leu Leu Leu Ser Asn Val Ser Pro Glu Leu Gly
            100                 105                 110

Gly Lys Tyr Tyr Phe Arg Gly Asp Leu Gly Gly Tyr Asn Gln Tyr Thr
        115                 120                 125

Phe Ser Glu His Ser Val Leu Asp Ile Val Asn Thr Pro Asn Ile Val
    130                 135                 140

Val Pro Pro Glu Val Val Ala Gly Thr Glu Val Glu Val Ser Cys Met
145                 150                 155                 160

Val Pro Asp Asn Cys Pro Glu Leu Arg Pro Glu Leu Ser Trp Leu Gly
                165                 170                 175

His Glu Gly Leu Gly Glu Pro Ala Val Leu Gly Arg Leu Arg Glu Asp
            180                 185                 190

Glu Gly Thr Trp Val Gln Val Ser Leu Leu His Phe Val Pro Thr Arg
        195                 200                 205

Glu Ala Asn Gly His Arg Leu Gly Cys Gln Ala Ser Phe Pro Asn Thr
```

```
    210                 215                 220

Thr Leu Gln Phe Glu Gly Tyr Ala Ser Met Asp Val Lys Tyr Pro Pro
225                 230                 235                 240

Val Ile Val Glu Met Asn Ser Ser Val Glu Ala Ile Glu Gly Ser His
                245                 250                 255

Val Ser Leu Leu Cys Gly Ala Asp Ser Asn Pro Pro Pro Leu Leu Thr
            260                 265                 270

Trp Met Arg Asp Gly Thr Val Leu Arg Glu Ala Val Ala Glu Ser Leu
        275                 280                 285

Leu Leu Glu Leu Glu Glu Val Thr Pro Ala Glu Asp Gly Val Tyr Ala
    290                 295                 300

Cys Leu Ala Glu Asn Ala Tyr Gly Gln Asp Asn Arg Thr Val Gly Leu
305                 310                 315                 320

Ser Val Met Tyr Ala Pro Trp Lys Pro Thr Val Asn Gly Thr Met Val
                325                 330                 335

Ala Val Glu Gly Glu Thr Val Ser Ile Leu Cys Ser Thr Gln Ser Asn
            340                 345                 350

Pro Asp Pro Ile Leu Thr Ile Phe Lys Glu Lys Gln Ile Leu Ser Thr
        355                 360                 365

Val Ile Tyr Glu Ser Glu Leu Gln Leu Glu Leu Pro Ala Val Ser Pro
    370                 375                 380

Glu Asp Asp Gly Glu Tyr Trp Cys Val Ala Glu Asn Gln Tyr Gly Gln
385                 390                 395                 400

Arg Ala Thr Ala Phe Asn Leu Ser Val Glu Phe Ala Pro Val Leu Leu
                405                 410                 415

Leu Glu Ser His Cys Ala Ala Ala Arg Asp Thr Val Gln Cys Leu Cys
            420                 425                 430

Val Val Lys Ser Asn Pro Glu Pro Ser Val Ala Phe Glu Leu Pro Ser
        435                 440                 445

Arg Asn Val Thr Val Asn Glu Ser Glu Arg Glu Phe Val Tyr Ser Glu
    450                 455                 460

Arg Ser Gly Leu Val Leu Thr Ser Ile Leu Thr Leu Arg Gly Gln Ala
465                 470                 475                 480

Gln Ala Pro Pro Arg Val Ile Cys Thr Ala Arg Asn Leu Tyr Gly Ala
                485                 490                 495

Lys Ser Leu Glu Leu Pro Phe Gln Gly Ala His Arg Leu Met Trp Ala
            500                 505                 510
```

```
Lys Ile Gly Pro Val Gly Ala Val Val Ala Phe Ala Ile Leu Ile Ala
        515                 520                 525

Ile Val Cys Tyr Ile Thr Gln Thr Arg Arg Lys Lys Asn Val Thr Glu
    530                 535                 540

Ser Pro Ser Phe Ser Ala Gly Asp Asn Pro Pro Val Leu Phe Ser Ser
545                 550                 555                 560

Asp Phe Arg Ile Ser Gly Ala Pro Glu Lys Tyr Glu Ser Lys Glu Val
                565                 570                 575

Ser Thr Leu Glu Ser His
            580
```

<210> 17
<211> 82
<212> DNA
<213> Artificial sequence

<220>
<223> Partial sequence of MOG f8

<400> 17

```
gttgcgactc ttgttcctat gctaagcttt gccgaggtgg gctggtatcg cagtccgttt     60

attgcagaag gtgatgaccc gg                                              82
```

<210> 18
<211> 200
<212> PRT
<213> Homo sapiens

<400> 18

```
Gly Gln Phe Arg Val Ile Gly Pro Arg His Pro Ile Arg Ala Leu Val
1               5                   10                  15

Gly Asp Glu Val Glu Leu Pro Cys Arg Ile Ser Pro Gly Lys Asn Ala
            20                  25                  30

Thr Gly Met Glu Val Gly Trp Tyr Arg Pro Pro Phe Ser Arg Val Val
        35                  40                  45

His Leu Tyr Arg Asn Gly Lys Asp Gln Asp Gly Asp Gln Ala Pro Glu
    50                  55                  60

Tyr Arg Gly Arg Thr Glu Leu Leu Lys Asp Ala Ile Gly Glu Gly Lys
65                  70                  75                  80

Val Thr Leu Arg Ile Arg Asn Val Arg Phe Ser Asp Glu Gly Gly Phe
                85                  90                  95

Thr Cys Phe Phe Arg Asp His Ser Tyr Gln Glu Glu Ala Ala Met Glu
            100                 105                 110

Leu Lys Val Glu Asp Pro Phe Tyr Trp Val Ser Pro Gly Val Leu Val
        115                 120                 125

Leu Leu Ala Val Leu Pro Val Leu Leu Leu Gln Ile Thr Val Gly Leu
    130                 135                 140

Val Phe Leu Cys Leu Gln Tyr Arg Leu Arg Gly Lys Leu Arg Ala Glu
145                 150                 155                 160

Ile Glu Asn Leu His Arg Thr Phe Glu Ser Phe Gly Val Leu Gly Pro
                165                 170                 175

Gln Val Lys Glu Pro Lys Lys Thr Gly Gln Phe Leu Glu Glu Leu Leu
            180                 185                 190

Phe His Leu Glu Ala Leu Ser Gly
        195                 200
```

<210> 19
<211> 8
<212> PRT
<213> Artificial sequence

<220>
<223> Human MOG 37-44 polypeptide

<400> 19

```
Val Gly Trp Tyr Arg Pro Pro Phe
1               5
```

<210> 20
<211> 9234
<212> DNA
<213> Artificial sequence

<220>
<223> Filamentous phage display vector f88-4

<400> 20

```
aacgctacta ccattagtag aattgatgcc accttttcag ctcgcgcccc aaatgaaaat      60
atagctaaac aggttattga ccatttgcga aatgtatcta atggtcaaac taaatctact     120
cgttcgcaga attgggaatc aactgttaca tggaatgaaa cttccagaca ccgtacttta     180
gttgcatatt taaaacatgt tgaactacag caccagattc agcaattaag ctctaagcca     240
tccgcaaaaa tgacctctta tcaaaaggag caattaaagg tactgtctaa tcctgacctg     300
ttggaatttg cttccggtct ggttcgcttt gaggctcgaa ttgaaacgcg atatttgaag     360
tctttcgggc ttcctcttaa tctttttgat gcaattcgct ttgcttctga ctataataga     420
cagggtaaag acctgatttt tgatttatgg tcattctcgt tttctgaact gtttaaagca     480
tttgaggggg attcaatgaa tatttatgac gattccgcag tattggacgc tatccagtct     540
aaacatttta caattacccc ctctggcaaa acttcctttg caaaagcctc tcgctatttt     600
ggtttctatc gtcgtctggt taatgagggt tatgatagtg ttgctcttac catgcctcgt     660
aattcctttt ggcgttatgt atctgcatta gttgagtgtg gtattcctaa atctcaattg     720
atgaatcttt ccacctgtaa taatgttgtt ccgttagttc gttttattaa cgtagatttt     780
tcctcccaac gtcctgactg gtataatgag ccagttctta aaatcgcata aggtaattca     840
aaatgattaa agttgaaatt aaaccgtctc aagcgcaatt tactacccgt tctggtgttt     900
ctcgtcaggg caagccttat tcactgaatg agcagctttg ttacgttgat ttgggtaatg     960
aatatccggt gcttgtcaag attactctcg acgaaggtca gccagcgtat gcgcctggtc    1020
```

tgtacaccgt gcatctgtcc tcgttcaaag ttggtcagtt cggttctctt atgattgacc 1080
gtctgcgcct cgttccggct aagtaacatg gagcaggtcg cggatttcga cacaatttat 1140
caggcgatga tacaaatctc cgttgtactt tgtttcgcgc ttggtataat cgctgggggt 1200
caaagatgag tgttttagtg tattctttcg cctctttcgt tttaggttgg tgccttcgta 1260
gtggcattac gtattttacc cgtttaatgg aaacttcctc atgaaaaagt ctttagtcct 1320
caaagcctcc gtagccgttg ctaccctcgt tccgatgctg tctttcgctg ctgagggtga 1380
cgatcccgca aaagcggcct ttgactccct gcaagcctca gcgaccgaat atatcggtta 1440
tgcgtgggcg atggttgttg tcattgtcgg cgcaactatc ggtatcaagc tgtttaagaa 1500
attcacctcg aaagcaagct gataaaccga tacaattaaa ggctcctttt ggagcctttt 1560
tttttggaga ttttcaacgt gaaaaaatta ttattcgcaa ttcctttagt tgttcctttc 1620
tattctcact ccgctgaaac tgttgaaagt tgtttagcaa aacctcatac agaaaattca 1680
tttactaacg tctggaaaga cgacaaaact ttagatcgtt acgctaacta tgagggctgt 1740
ctgtggaatg ctacaggcgt tgtggtttgt actggtgacg aaactcagtg ttacggtaca 1800
tgggttccta ttgggcttgc tatccctgaa aatgagggtg gtggctctga gggtggcggt 1860
tctgagggtg gcggttctga gggtggcggt actaaacctc ctgagtacgg tgatacacct 1920
attccgggct atacttatat caaccctctc gacggcactt atccgcctgg tactgagcaa 1980
aaccccgcta atcctaatcc ttctcttgag gagtctcagc ctcttaatac tttcatgttt 2040
cagaataata ggttccgaaa taggcagggt gcattaactg tttatacggg cactgttact 2100
caaggcactg accccgttaa aacttattac cagtacactc ctgtatcatc aaaagccatg 2160
tatgacgctt actggaacgg taaattcaga gactgcgctt tccattctgg ctttaatgag 2220
gatccattcg tttgtgaata tcaaggccaa tcgtctgacc tgcctcaacc tcctgtcaat 2280
gctggcggcg gctctggtgg tggttctggt ggcggctctg agggtggcgg ctctgagggt 2340
ggcggttctg agggtggcgg ctctgagggt ggcggttccg gtggcggctc cggttccggt 2400
gattttgatt atgaaaaaat ggcaaacgct aataaggggg ctatgaccga aaatgccgat 2460
gaaaacgcgc tacagtctga cgctaaaggc aaacttgatt ctgtcgctac tgattacggt 2520
gctgctatcg atggtttcat tggtgacgtt tccggccttg ctaatggtaa tggtgctact 2580
ggtgattttg ctggctctaa ttcccaaatg gctcaagtcg gtgacggtga taattcacct 2640
ttaatgaata atttccgtca atatttacct tctttgcctc agtcggttga atgtcgccct 2700
tatgtctttg gcgctggtaa accatatgaa ttttctattg attgtgacaa aataaactta 2760
ttccgtggtg tctttgcgtt tcttttatat gttgccacct ttatgtatgt attttcgacg 2820
tttgctaaca tactgcgtaa taaggagtct taatcatgcc agttcttttg ggtattccgt 2880
tattattgcg tttcctcggt ttccttctgg taactttgtt cggctatctg cttactttcc 2940
ttaaaaaggg cttcggtaag atagctattg ctatttcatt gtttcttgct cttattattg 3000
ggcttaactc aattcttgtg ggttatctct ctgatattag cgcacaatta ccctctgatt 3060
ttgttcaggg cgttcagtta attctcccgt ctaatgcgct tccctgtttt tatgttattc 3120
tctctgtaaa ggctgctatt ttcatttttg acgttaaaca aaaaatcgtt tcttatttgg 3180
attgggataa ataaatatgg ctgtttattt tgtaactggc aaattaggct ctggaaagac 3240
gctcgttagc gttggtaaga ttcaggataa aattgtagct gggtgcaaaa tagcaactaa 3300

```
tcttgattta aggcttcaaa acctcccgca agtcgggagg ttcgctaaaa cgcctcgcgt   3360
tcttagaata ccggataagc cttctatttc tgatttgctt gctattggtc gtggtaatga   3420
ttcctacgac gaaaataaaa acggtttgct tgttcttgat gaatgcggta cttggtttaa   3480
tacccgttca tggaatgaca aggaaagaca gccgattatt gattggtttc ttcatgctcg   3540
taaattggga tgggatatta tttttcttgt tcaggattta tctattgttg ataaacaggc   3600
gcgttctgca ttagctgaac acgttgttta ttgtcgccgt ctggacagaa ttactttacc   3660
ctttgtcggc actttatatt ctcttgttac tggctcaaaa atgcctctgc ctaaattaca   3720
tgttggtgtt gttaaatatg gtgattctca attaagccct actgttgagc gttggcttta   3780
tactggtaag aatttatata acgcatatga cactaaacag gctttttcca gtaattatga   3840
ttcaggtgtt tattcatatt taacccctta tttatcacac ggtcggtatt tcaaaccatt   3900
aaatttaggt cagaagatga aattaactaa aatatatttg aaaaagtttt ctcgcgttct   3960
ttgtcttgcg ataggatttg catcagcatt tacatatagt tatataaccc aacctaagcc   4020
ggaggttaaa aaggtagtct ctcagaccta tgattttgat aaattcacta ttgactcttc   4080
tcagcgtctt aatctaagct atcgctatgt tttcaaggat tctaagggaa aattaattaa   4140
tagcgacgat ttacagaagc aaggttattc catcacatat attgatttat gtactgtttc   4200
aattaaaaaa ggtaattcaa atgaaattgt taaatgtaat taattttgtt ttcttgatgt   4260
ttgtttcatc atcttctttt gctcaagtaa ttgaaatgaa taattcgcct ctgcgcgatt   4320
tcgtgacttg gtattcaaag caaacaggtg aatctgttat tgtctcacct gatgttaaag   4380
gtacagtgac tgtatattcc tctgacgtta agcctgaaaa tttacgcaat ttctttatct   4440
ctgttttacg tgctaataat tttgatatgg ttggctcaat tccttccata attcagaaat   4500
ataacccaaa tagtcaggat tatattgatg aattgccatc atctgatatt caggaatatg   4560
atgataattc cgctccttct ggtggtttct ttgttccgca aaatgataat gttactcaaa   4620
catttaaaat taataacgtt cgcgcaaagg atttaataag ggttgtagaa ttgtttgtta   4680
aatctaatac atctaaatcc tcaaatgtat tatctgttga tggttctaac ttattagtag   4740
ttagcgcccc taaagatatt ttagataacc ttccgcaatt tctttctact gttgatttgc   4800
caactgacca gatattgatt gaaggattaa ttttcgaggt tcagcaaggt gatgctttag   4860
atttttcctt tgctgctggc tctcagcgcg gcactgttgc tggtggtgtt aatactgacc   4920
gtctaacctc tgttttatct tctgcgggtg gttcgttcgg tatttttaac ggcgatgttt   4980
tagggctatc agttcgcgca ttaaagacta atagccattc aaaaatattg tctgtgcctc   5040
gtattcttac gctttcaggt cagaagggtt ctatttctgt tggccagaat gtccctttta   5100
ttactggtcg tgtaactggt gaatctgcca atgtaaataa tccatttcag acggttgagc   5160
gtcaaaatgt tggtatttct atgagtgttt ttcccgttgc aatggctggc ggtaatattg   5220
ttttagatat aaccagtaag gccgatagtt tgagttcttc tactcaggca agtgatgtta   5280
ttactaatca aagaagtatt gcgacaacgg ttaatttgcg tgatggtcag actcttttgc   5340
tcggtggcct cactgattac aaaaacactt ctcaagattc tggtgtgccg ttcctgtcta   5400
aaatcccttt aatcggcctc ctgtttagct cccgttctga ttctaacgag gaaagcacgt   5460
tgtacgtgct cgtcaaagca accatagtac gcgccctgta gcggcgcatt aagcgcggcg   5520
```

ggtgtggtgg ttacgcgcag cgtgaccgct acacttgcca gcgccctagc gcccgctcct 5580

ttcgctttct tcccttcctt tctcgccacg ttctccggct ttccccgtca agctctaaat 5640

cgggggagct cgagcttact ccccatcccc ctgttgacaa ttaatcatcg gctcgtataa 5700

tgtgtggaat tgtgagcgga taacaatttc ttaatggaaa cttcctcatg aaaaagtctt 5760

tagttcttaa agcatctgtt gctgttgcga ctcttgttcc tatgctaagc tttgccaacg 5820

tccctgcaga aggtgatgac ccggctaaag ctgcttttga ctctcttcag gcttctgcta 5880

ctgaatacat cggctacgct tgggctatgg tggttgttat cgttggtgct actattggca 5940

tcaaactttt caaaaaattc acttctaaag cgtcttaatg aactcagata cccagcccgc 6000

ctaatgagcg ggcttttttt taagctagct tatgatgatg atgtgcttaa aaacttactc 6060

aatggctggt ttatgcatat cgcaatacat gcgaaaaacc taaaagagct tgccgataaa 6120

aaaggccaat ttattgctat ttaccgcggc tttttattga gcttgaaaga taaataaaat 6180

agataggttt tatttgaagc taaatcttct ttatcgtaaa aaatgccctc ttgggttatc 6240

aagagggtca ttatatttcg cggaataaca tcatttggtg acgaaataac taagcacttg 6300

tctcctgttt actcccctga gcttgagggg ttaacatgaa ggtcatcgat agcaggataa 6360

taatacagta aaacgctaaa ccaataatcc aaatccagcc atcccaaatt ggtagtgaat 6420

gattataaat aacagcaaac agtaatgggc caataacacc ggttgcattg gtaaggctca 6480

ccaataatcc ctgtaaagca ccttgctgat gactctttgt ttggatagac atcactccct 6540

gtaatgcagg taaagcgatc ccaccaccag ccaataaaat taaaacaggg aaaactaacc 6600

aaccttcaga tataaacgct aaaaaggcaa atgcactact atctgcaata aatccgagca 6660

gtactgccgt tttttcgccc catttagtgg ctattcttcc tgccacaaag gcttggaata 6720

ctgagtgtaa aagaccaaga cccgctaatg aaaagccaac catcatgcta ttccatccaa 6780

aacgattttc ggtaaatagc acccacaccg ttgcgggaat ttggcctatc aattgcgctg 6840

aaaaataaat aatcaacaaa atgggcatcg ttttaaataa agtgatgtat accgaattcg 6900

attgcgtctc aacccctact tcggtatctg tattatcacg tgtatttttg gtttcacgga 6960

accaaaacat aaccacaagg aaagtgacaa tatttagcaa cgcagcgata aaaaagggac 7020

tatgcggtga aatctctcct gcaaaaccac caataatagg ccccgctatt aaaccaagcc 7080

caaaacttgc ccctaaccaa ccgaaccact tcacgcgttg agaagctgag gtggtatcgg 7140

caatgaccga tgccgcgaca gccccagtag ctcctgtgat ccctgaaagc aaacggccta 7200

aatacagcat ccaaagcgca cttgaaaaag ccagcaataa gtaatccagc gatgcgccta 7260

ttaatgacaa caacagcact gggcgccgac caaatcggtc agacattttt ccaagccaag 7320

gagcaaagat aacctgcatt aacgcataaa gtgcaagcaa tacgccaaag tggttagcga 7380

tatcttccga agcaataaat tcacgtaata acgttggcaa gactggcatg ataaggccaa 7440

tccccatggc atcgagtaac gtaattacca atgcgatctt tgtcgaacta ttcatttcac 7500

ttttctctat cactgatagg gagtggtaaa ataactctat caatgataga gtgtcaacaa 7560

aaattaggaa ttaatgatgt ctagattaga taaaagtaaa gtgattaaca gcgcattaga 7620

gctgcttaat gaggtcggaa tcgaaggttt aacaacccgt aaactcgccc agaagctagg 7680

tgtagagcag cctacattgt attggcatgt aaaaaataag cgggctttgc tcgacgcctt 7740

agccattgag atgttagata ggcaccatac tcacttttgc cctttagaag gggaaagctg 7800

```
gcaagatttt ttacgtaata acgctaaaag ttttagatgt gctttactaa gtcatcgcga    7860
tggagcaaaa gtacatttag gtacacggcc tacagaaaaa cagtatgaaa ctctcgaaaa    7920
tcaattagcc tttttatgcc aacaaggttt ttcactagag aatgcattat atgcactcag    7980
cgctgtgggg cattttactt taggttgcgt attggaagat caagagcatc aagtcgctaa    8040
agaagaaagg gaaacaccta ctactgatag tatgccgcca ttattacgac aagctatcga    8100
attatttgat caccaaggtg cagagccagc cttcttattc ggccttgaat tgatcatatg    8160
cggattagaa aaacaactta aatgtgaaag tgggtcttaa aagcagcata acctttttcc    8220
gtgatggtaa cttcacggta accaagatgt cgagttaacc accctttaga ttcataaagc    8280
gaaaataatg cggctccaac gtacccacct aaatggaaac ggcgttcact ccaatctaaa    8340
cacgcacaac agattttacg tgaatgtttg gaaggaacgt caattcccat ttcatgaaaa    8400
tattgaatac cacttaatgt gatcattgaa ccattttcag tgatccattg ctgttgacaa    8460
agggaatcat agatcccttt agggttccga tttagtgctt tacggcacct cgacctccaa    8520
aaacttgatt tgggtgatgg ttcacgtagt gggccatcgc cctgatagac ggtttttcgc    8580
cctttgacgt tggagtccac gttctttaat agtggactct tgttccaaac tggaacaaca    8640
ctcacaacta actcggccta ttcttttgat ttataaggat tttgtcatt  ttctgcttac    8700
tggttaaaaa ataagctgat ttaacaaata tttaacgcga aatttaacaa aacattaacg    8760
tttacaattt aaatatttgc ttatacaatc atcctgtttt tggggctttt ctgattatca    8820
atcggggtac atatgattga catgctagtt ttacgattac cgttcatcga ttctcttgtt    8880
tgctccagac tttcaggtaa tgacctgata gcctttgtag acctctcaaa aatagctacc    8940
ctctccggca tgaatttatc agctagaacg gttgaatatc atattgacgg tgatttgact    9000
gtctccggcc tttctcaccc gtttgaatct ttgcctactc attactccgg cattgcattt    9060
aaaatatatg agggttctaa aaatttttat ccctgcgttg aaattaaggc ttcaccagca    9120
aaagtattac agggtcataa tgtttttggt acaaccgatt tagctttatg ctctgaggct    9180
ttattgctta attttgctaa ctctctgcct tgcttgtacg atttattgga tgtt          9234
```

<210> 21
<211> 24
<212> DNA
<213> Artificial sequence

<220>
<223> MOG 37-44 epitope coding sequence

<400> 21
gtggggtggt accgcccccc cttc 24

<210> 22
<211> 77
<212> DNA
<213> Artificial sequence

<220>
<223> Partial sequence of MOG f88

<400> 22

```
accctcgttc cgatgctgtc tttcgctgca gaggtgggct ggtatcgcag tccgtttgag     60
gatcccgcaa aagcggc                                                    77
```